# EUROPEAN PATENT APPLICATION

(11) **EP 2 668 955 A2**
(43) Date of publication of application: **04.12.2013**
(21) Application number: 12739036.7
(22) Date of filing: 30.01.2012
(51) Int. Cl.: A61K 36/234, A61K 36/704, A61P 11/06, A61P 11/00

(54) **COMPOSITION FOR PREVENTING OR TREATING A RESPIRATORY DISEASE CONTAINING A MIXED HERBAL EXTRACT OF CNIDIUM OFFICINALE ROOT AND POLYGONI CUSPIDATI ROOT**

(30) Priority: 28.01.2011 KR 20110009114; 20.01.2012 KR 20120006849
(71) Applicant: Whanin Pharmaceutical Co., Ltd., Seoul 443-270 (KR)
(72) Inventor: CHO, Yong Baik, Uiwang-si Gyeonggi-do 437-758 (KR); KANG, Moon Kyu, Namyangju-si Gyeonggi-do 472-080 (KR); JUNG, In Ho, Suwon-Si Gyeonggi-do 440-837 (KR); HUR, Jong Hyun, Suwon-si Gyeonggi-do 443-751 (KR); KIM, Soon Han, Suwon-si Gyeonggi-do 442-190 (KR); JUNG, Kyoung Chul, Suwon-si Gyeonggi-do 441-400 (KR); LEE, Je Young, Seoul 143-817 (KR); KIM, Seul Ki, Suwon-si Gyeonggi-do 442-190 (KR); LEE, Jee Young, Yongin-si Gyeonggi-do 446-735 (KR); YU, Byung Chul, Yongin-si Gyeonggi-do 446-740 (KR); HYON, Min-Kyong, Seoul 138-786 (KR)
(74) Representative: Albrecht, Thomas
(86) International application number: PCT/KR2012/000650
(87) International publication number: WO 2012/102581

(57) **Abstract**

The present invention relates to a composition for preventing or treating a respiratory disease containing an active ingredient in the form of a mixed herbal extract of Cnidii Rhizoma and Polygoni cuspidati Radix, and relates to a production method therefor. The mixed herbal extract of Cnidii Rhizoma and Polygoni cuspidati Radix has an outstanding 5-lipoxygenase suppressing activity, airway constriction suppressing activity, airway inflammation suppressing action, ear-swelling anti-inflammatory effect and antitussive and phlegm-loosening action, and has been confirmed to be useful in preventing or treating respiratory diseases including asthma, chronic obstructive pulmonary disease, acute and chronic bronchitis, allergic rhinitis, cough, expectoration, acute lower respiratory infections (bronchitis and bronchiolitis), and acute upper respiratory infections such an pharyngitis, tonsillitis and laryngitis.

## Description

### Technical Field

The present invention relates to a composition for preventing or treating respiratory disease containing an extract of a mixture of Cnidii rhizoma and Polygoni cuspidati radix as an active ingredient, and more particularly to a composition for preventing or treating respiratory diseases, including asthma, chronic obstructive pulmonary disease, acute and chronic bronchitis, allergic rhinitis, coughing, phlegm, acute lower respiratory infections (bronchitis and bronchiolitis), and acute lower respiratory infections such as laryngopharyngitis, tonsillitis and laryngitis, the composition containing an extract of a mixture of Cnidii rhizoma and Polygoni cuspidati radix, which was found to have excellent 5-lipoxygenase inhibitory activity, airway constriction inhibitory activity, airway inflammation inhibitory activity, ear edema/inflammation inhibitory activity and antitussive and expectorant activities.

### Background Art

Asthma, chronic obstructive pulmonary disease, allergic nasitis, coughing, phlegm, acute and chronic bronchitis, bronchiolitis, sore throat (pharyngitis), tonsillitis, laryngitis and the like are typical respiratory diseases.

The term "asthma" refers to chronic inflammation in the airway, particularly, bronchi. Inflammation caused by asthma can be worsened by very various causes, including smoke, allergic antigens, cold wind and respiratory infection, and when this inflammation continues, it causes airway deformation and airway hyper-responsiveness. Due to such causes, general symptoms appear, including wheezing (a symptom of airway narrowing that shows dry or feeble breathing), short breathing, coughing, and excessive expectoration.

Asthma can be characterized by four pathological symptoms, including a significant increase in the entry of eosinophils into the airway, the excessive secretion of mucus, the observation of edema, and narrowing of the airway.

The respiratory airway consists mainly of mucosa and bronchial smooth muscles. The mucosa has many glands that continuously secrete necessary secretions, and as the bronchial smooth muscles contract, the respiratory airway becomes narrower. When an inflammation reaction in the airway is caused by very various causes, including smoke, allergic antigens, cold wind and respiratory infection, secretions from the glands further increase to obstruct the airway, so that the glands swell up to make the airway narrower. For this reason, spasmodic coughing and severe breathing difficulty accompanied by wheezing appear, and upon spasmodic coughing, dry coughing occurs and chest pain is felt. In addition, asthma having only difficult breathing symptoms with chronic coughing and chest pain without wheezing is also frequent, and such symptoms tend to appear spasmodically during everyday life.

Recently, asthma was newly defined as a chronic bronchial inflammatory disease rather than bronchial stenosis. When asthma symptoms appear, a fundamental therapeutic method for controlling inflammation over a long period of time is important, even though it is also important to alleviate the symptoms.

When viewed in pathological/physiological terms, asthma is a disease manifested by airway inflammation, airway-hyperresponsiveness (AHR), and mucin hypersecretion, and when viewed in immunological terms, asthma is a chronic airway inflammatory disease characterized by the invasion of eosinophils, an increase in Th2 cells compared to Th1 cells, and an increase in activated mast cells. Asthma is typically characterized by the invasion of eosinophilic granulocytes (leukocytes) into the airway, and the eosinophils in asthma perform a very important role in the pathology/pathology of asthma by producing various inducers that promote airway inflammation and bronchial constriction. Antigens that cause asthma cause the differentiation of T cells into Th2 cells, in which the Th2 cells secrete cytokines, including IL (interleukin)-5, GM-CSF (granulocyte-macrophage stimulating factor), IL-3, IL-13, IL-4 and the like, in which L-4 acts on B cells to promote the production of IgE and activates mast cells. When inflammatory cells such as mast cells are activated, they release various inflammation mediators and cause acute inflammatory reactions, including bronchial constriction, vasodilation, sensory nerve sensitization, and cholinergic bronchoconstriction, in the airway. Most asthma symptoms are reversible, but in some asthma patients, as asthma progresses, the fibrosis of epithelial cells, an increase in the number of blood vessels and mucus cells, and the hypertrophy of bronchial smooth cells occur so that the airway structure is changed and remodeled. In addition, airway obstruction that occurs in chronic obstructive pulmonary disease (COPD) may occur.

The term "chronic obstructive pulmonary disease" (COPD) refers to a disease group in which airway obstruction occurs without causative pulmonary disease and heart disease to reduce the rate of air flow. Clinically, chronic obstructive pulmonary disease collectively includes chronic bronchitis in which coughing accompanied by phlegm occurs, and pulmonary emphysema in which alveoli below terminal bronchioles abnormally increase and the alveolar septa, in which the chronic bronchitis and the pulmonary emphysema are mixed to be difficult to distinguish from each other. Chronic obstructive pulmonary disease shows airway disease symptoms, including difficult breathing, coughing, phlegm and the like, like asthma, and then worsens pulmonary function, eventually leading to death. It is mainly caused by smoking, environmental pollution, respiratory infection and the like, and is known to be induced by neutrophils and macrophages. Neutrophils are typical inflammatory cells and secrete various proteases to cause lung parenchymal destruction and chronic mucus secretion, and macrophages were recently reported to be important inflammatory cells that are involved in the overexpression of IFN-γ (interferon-γ) and IL-13. In addition, macrophages secrete mediators that cause damage to tissue, including ROS (reactive oxygen species), NO (nitric oxide) metabolites and the like, and secrete mediators that are involved in wound healing, including TGF-β (transforming growth factor-β), FGF2 (fibroblast growth factor 2), VEGF (endothelial growth factor) and the like. Thus, macrophages are the major cause of chronic inflammation. Irreversible airway obstructions include pulmonary emphysema which is caused by airway obstruction due to the destruction of alveoli, small airway fibrosis caused by bronchiolitis leading to repeated injury, and obstructive bronchiolitis. Such diseases are mostly accompanied by small airway inflammation and fibrosis together with pulmonary emphysema.

Asthma and chronic obstructive pulmonary disease are common in that they show airway disease symptoms, including difficult breathing, coughing, phlegm and the like and there is no agent for treating the causes of these diseases. Only two agents, including a controller for inhibiting inflammation and a reliever for relieving difficult breathing symptoms, are currently used to treat asthma, and an agent for treating the cause of asthma has not yet been reported. This is because asthma is caused by very various factors and inducers, but the apparent symptoms of asthma are similar to each other. In addition, this is believed to be because studies on the cause of asthma are difficult. Particularly, high-dose steroid therapy is used to treat severe persistent asthma that forms a significant portion of asthma-related total mortality and economic burden, but this therapy still has a problem in that asthma is not easily controlled. Asthma treatment should be performed by a combination of drug therapy, environmental therapy (avoidance therapy) and immunotherapy, and when asthma remains untreated for a long period of time, the bronchial mucosa is damaged, and this damage cannot be healed and frequently worsens asthma. For this reason, it is important to treat asthma in an early stage, and the thorough treatment of asthma is required, because inflammation of the bronchial mucosa continues to cause bronchial damage, even though asthma symptoms disappear. Asthma is chronic, can recur after temporary alleviation and can become worse even during treatment. For this reason, particularly, asthma in childhood is required to be actively treated in an early stage to reduce the number and degree of asthma attacks in order to make it easy to perfectly cure asthma. Because asthma is an allergic disease, it is not easy to treat, and unless asthma is fundamentally treated, it easily recurs when being exposed to an asthma-inducing environment, and for this reason, thorough environmental controls, including drug therapy, are required.

Another typical respiratory disease is allergic rhinitis, also called "nasal allergy". The symptoms of allergic rhinitis include continuous sneezing that occurs suddenly, a large amount of clear snivel, nasal congestion, heavy head feeling, and tearing. Rhinitis, the antigen of which is unclear while having symptoms very similar to the symptoms of allergic rhinitis, is referred to as vasomotor rhinitis. Specifically, in the case of allergic rhinitis, symptoms as described above appear when the body is temporarily chilled after rising in the morning, and then the symptoms disappear within several hours. The symptoms of allergic rhinitis often occur during a change of season or a cold season. Allergic rhinitis is often confused with coryza, but differs from a cold and is frequently accompanied by asthma or urticaria.

Allergic rhinitis mediates an antigen-antibody hypersensitivity reaction, an initial reaction, which appears between 2 and 90 minutes after exposure to an antigen and in which histamine and arachidonic acid are released from the membrane of mast cells and basophils so that prostaglandins and leukotrienes are produced by cyclooxygenase (COX) and 5-lipoxygenase (5-LO), and a late reaction which appears 4-8 hours after exposure to the antigen. The initial reaction is mainly caused by mediators, and the late reaction is mainly caused by the invasion of cells. In addition, allergic rhinitis and non-allergic rhinitis all act as risk factors that cause asthma. Treatment of allergic rhinitis is performed by desensitization therapy when the antigen thereof is clear. Other methods for the treatment of allergic rhinitis include drug therapy, surgical therapy and physical therapy, but it is difficult to perfectly cure allergic rhinitis.

Pyothorax is a phenomenon in which pus is gathered in the pleural space surrounding the lung and the thorax. It is mainly caused by pneumonia due to *Staphylococcus aureus, Streptococcus pneumoniae* and influenza virus, which invade the lung and spread to the pleural space.

Coughing and phlegm are caused by physical and chemical factors such as cold air, external foreign materials including pathogenic microorganisms, air pollutants, and allergy-causing materials. Coughing is a defense mechanism that occurs in response to stimulation of the airway mucosa, and when continuous coughing is caused by excessive stimulation of the airway mucosa, it reduces the quality of life of the patient. Like the case of coughing, when external dust or stimuli are introduced into the body, the bronchus removes these introduced materials together with saliva by muscular movement, and these materials cause the formation of phlegm, and thick purulent phlegm is produced by bronchial inflammation. Herein, inhibition of coughing is referred to as antitussive, and inhibition of phlegm is referred to as expectoration.

Acute bronchitis is not an independent disease, but is accompanied by diseases of the upper and lower airways. Acute bronchitis occurs together with upper airway infection such as nasopharyngitis, influenza, pertussis, measles, typhoid fever, diphtheria, and scarlet fever, and is mainly caused by viral infection. In the case of acute bronchitis, various types of Streptococcus, including *Streptococcus pneumoniae, Staphylococcus aureus,* and Haemophilus influenza, are often found in phlegm, but it does not means that acute bronchitis is caused by bacterial infection, and antibiotic therapy does not influence the progress of the disease. The main symptoms of acute bronchitis include redness, swelling and dryness in the bronchial mucosa, and mucopurulent secretions in the bronchus. It is commonly cured without causing complications, but when it progresses to chronic bronchitis, it causes a swelling, hypertrophy and atrophy in the mucosa. When it remains untreated for a long period of time, it causes fibrous tissue proliferation, bronchial stenosis or emphysema.

Chronic bronchitis is a disease which continues for 2 years or more and in which phlegm and coughing continue over 3 months or more every year. It is presumed to be caused by bronchial damage due to stimuli, including smoking, air pollution and occupational exposure, and the main symptoms thereof include chronic coughing, phlegm, and difficult breathing in exercise. When the disease progresses, difficult breathing becomes more severe over several months to several years, and thus the patient feels difficult breathing even upon slight activity.

Laryngitis is caused by viral or bacterial infection of the larynx or the spreading of inflammation of the surrounding tissue, such as pharyngitis or tonsillitis, to the larynx. It often appears as a symptom of a cold and is generally accompanied by coryza (acute rhinitis) or pharyngitis, coughing, and a change in a voice. Laryngitis is an infectious disease commonly designated as a respiratory disease of the upper airway and is difficult to clearly distinguish from pharyngitis and bronchitis.

Bronchiolitis is an inflammation of the alveolus, which causes difficulty in breathing. When bronchiolitis occurs, inflammation occurs on the inner wall of the bronchiole, so that cells of the inner wall lump together or swell to narrow the lumen, resulting in difficulty in breathing. Bronchiolitis is contagious and frequently epidemic. The bronchiole functions to transfer air from the bronchus to the alveolus, and the alveolus functions to supply oxygen to blood. Bronchiolitis is caused by infection with viruses and/or bacteria. In some cases, bronchiolitis occurs whenever a cold occurs. Risk factors that cause bronchiolitis include diseases that reduces resistance, particularly, respiratory infection and allergic diseases, family history, obesity, etc. Complications of bronchiolitis include chronic lung diseases, including chronic bronchitis, partial collapse of the lung, bronchiectasis, recurrence of pneumonia, and chronic obstructive pulmonary disease in rare cases.

Laryngopharyngitis is a disease corresponding to a common cold, sore throat or upper airway infection. It refers to an inflammation of the upper airway mucosa (including the pharynx and the larynx), which is caused by infection with bacteria such as beta-hemolytic Streptococcus, Staphylococcus, Pneumococcus, Haemophilus, and anaerobic bacteria, or viruses such as influenza virus, herpes simplex virus, para-influenza virus, Coxsackie virus, echovirus and the like. Laryngopharyngitis is classified into acute laryngopharyngitis and chronic laryngopharyngitis. Acute laryngopharyngitis is caused by a sudden change in temperature, a cold, recessive disease, overwork, weak constitution, or bacterial infection. Chronic laryngopharyngitis is caused by repeated recurrence of laryngopharyngitis, excessive smoking, drinking, overwork, intake of pungent food, exploitation of a throat, reflux disease of the throat, etc. In rare cases, it can be caused by the inhalation of irritant gas, chemicals or chemical vapor or the spreading of inflammation from adjacent sites such as accessory sinuses.

As described above, respiratory diseases, including asthma, chronic obstructive pulmonary disease, allergic rhinitis, coughing, phlegm, acute and chronic bronchitis, bronchiolitis, laryngopharyngitis, tonsillitis and laryngitis, have some differences in the causes and symptoms thereof, but are common in that they are inflammatory diseases. Among drugs which are currently used, airway relaxants merely relieve symptoms without being effective against inflammation that worsens diseases, and for this reason, when the airway relaxants are used for a long period of time, they can cause drug resistance to worsen diseases. In addition, steroidal drugs known to be effective against inflammation have serious side effects, and thus are problematic upon long-term use. For this reason, the two types of drugs are frequently formulated in combination, and in this case, these drugs are formulated in the form of inhalation preparations rather than oral preparations due to the side effects of the steroids, and thus are difficulty to take, and have a low compliance.

Thus, there is an urgent need for the development of new therapeutic agents which can overcome these limitations of conventional therapeutic drugs to fundamentally treat the causes of diseases and effectively relieve symptoms. However, as described above, various leukocytes and various cytokines and inflammation mediators, which are released from the leukocytes, are involved in respiratory diseases, and thus it is believed that respiratory diseases are difficult to effectively treat with synthetic medical drugs having single components and that herbal extracts having various components and mechanisms can be effective therapeutic agents for respiratory diseases.

In this viewpoint, the present invention provides a composition for preventing or treating respiratory diseases, which contains an extract of a mixture of Cnidii rhizoma and Polygoni cuspidati radix.

Cnidii rhizoma (*Cnidium Officinale* Root) is a medicinal resource plant that uses the rhizome of *Ligusticum chuanxiong* Hort. or *Cnidium officinale* Makino and is a perennial plant belonging to the family Umbelliferae. The known main components of Cnidii rhizoma include essential oil components, including cnidilide, z-ligustilide, butylidenephthalide and senkyunolide (Chem. Pharm. Bull., 1984, 32, 3770-3773; Korean J. Phamacogn. 1990, 21, 69-73), and phenolic compounds, including ferulic acid, chlorogenic acid and the like (Yakugaku zasshi, 1989, 109, 402-406). Cnidii rhizoma is known to have pharmacological effects, including antismasmodic, sedative, blood pressure lowering, vasidilation, antimicrobial and antifungal effects (Korean Society Tabacco Sci., 1994, 16, 20-25) and is used for diseases, including headache, sterility, menstrual irregularity, tonic, cold hypersensitivity, and anemia, in Chinese medicine (Modern Pharmacognosy, Society for the research of pharmacognosy, 1994, pp. 338-340, Hakchangsa).

Polygoni cuspidati radix (*Polygoni Cuspidati* Root) is the root of *Polygonum cuspidatim* Siebold et Zuccarinii or other plants belonging to the same genus and is a perennial plant belonging to the family *Polygonaceae.* The known main components of *Polygoni cuspidati* radix include anthraquinone derivatives, including emodin, physcion and chrysophanol, and stilbene derivatives, including resveratrol (Free Radic Res., 32, 2000, 135-44). *Polygoni cuspidati* radix was reported to have pharmacological effects, including anti-myocardial Ischemia, anti-inflammatory, antioxidant, anti-cholesterol and anticancer effects (Antiviral Research, 2005, 66, 29-34) and is known in Chinese medicine to have effects on palsy elimination, dampness elimination, extravasated blood spread, pain elimination, antitussive and expectorant effects (Herbiology, 1998, 420-421, Younglimsa).

In addition, the inventive extract of a mixture of Cnidii rhizoma and Polygoni cuspidati radix has an activity of inhibiting 5-lipoxygenase (5-LO) activity, and thus can be used for the prevention or treatment of respiratory diseases. 5-lipoxygenase is known as a major enzyme required for producing leukotrienes (LTs) from arachidonic acid (Science, 1983, 220, 568-75). The produced leukotrienes are known as major factors that cause airway constriction and inflammation, which are serious symptoms appearing in respiratory diseases, including asthma and bronchitis, and thus it is believed that the inventive extract can inhibit 5-lipoxygenase to effectively block the production of leukotrienes so as to relieve airway constriction and inflammation, thereby effectively treating respiratory diseases.

A process in which leukotrienes are produced by 5-lipoxygenase is as follows. First, phospholipase A2 is activated by the IgE-mediated activation of mast cells (immune cells that are the main cause of allergy, and the surface of mast cells has factors to which IgE type antibody can bind) in the respiratory airway and produces arachidonic acid from phospholipids on the cell membrane. Also, the mast cells are stimulated so that 5-lipoxygenase is transferred from the cytoplasm to the nuclear membrane, and the transferred 5-lipoxygenase is activated by 5-lipoxygenase activating protein in the nuclear membrane (Science, 1983, 220, 568-75). The activated 5-lipoxygenase oxygenates arachidonic acid to produce HPETE (hydroperoxyeicosatetraenoic acid), and the produced HPETE is converted to LTA4 (leukotriene A4) by LTA synthase (leukotriene A synthase), and then converted to LTB4 (leukotriene B4) or LTC4 (leukotriene C4) (Science, 1983, 220, 568-75).

LTA4 (leukotriene A4) is converted to LTB4 by LTA4 hydroxylase (leukotriene A4 hydroxylase) or converted to LTC4 (leukotriene C4) by LTC4 (leukotriene C4) synthase. The produced LTC4 (leukotriene C4) migrates to the outside of the cells and is metabolized into LTD4 (leukotriene D4) and LTE4 (leukotriene E4) (Science, 1983, 220, 568-75).

LTB4 (leukotriene B4) is a potent chemoattractant for neutrophils, eosinophils, monocytes and the like and is known as a major factor that causes airway inflammation by attaching phagocytes to the blood vessel wall, degranulating neutrophils and producing superoxide anions. LTC4 (leukotriene C4) and its metabolite LTD4 (leukotriene D4) is known as a potent bronchoconstrictor that increases vascular permeability and the mucus secretion of the airway (Science, 1983, 220, 568-75).

LTC4 (leukotriene C4), LTD4 (leukotriene D4) and LTE4 (leukotriene E4) all have cysteine residues, and thus are collectively referred to as cysLTs. These leukotrienes are mostly produced in mast cells, eosinophils and alveolar macrophages and are known as major factors that act on cysLT1 (cysLT receptor type 1) to stimulate airway smooth muscles to cause airway constriction, excessive mucus secretion, and inflammatory reactions in respiratory diseases such as asthma (Science, 1983, 220, 568-75).

Meanwhile, it is known that respiratory diseases can be treated by inhibiting 5-lipoxygenase to effectively inhibit the production of leukotrienes so as to inhibit airway constriction and excessive mucus secretion and relieve inflammation (Curr. Med. Chem., 2007, 14, 1966-77; Prostaglandins & Lipid Mediators, 2007, 83, 188-97; Pediatrics 2008, 122, pp. 1249-55). Accordingly, the present inventors have found that an extract of a mixture of Cnidii rhizoma and Polygoni cuspidati radix has significantly high 5-liposygenase inhibitory activity, airway constriction inhibitory activity, airway inflammation inhibitory activity, ear edema/inflammation inhibitory activity, and antitussive and expectorant activities, compared to an extract of each of the plants, thereby completing the present invention.

Korean Patent Publication No. 2006-128153 discloses that an ethanol extract of Cnidii rhizoma exhibits an anti-inflammatory effect by inhibiting 5-lipoxygenase activity, but the use of the extract is limited to cosmetic products. In addition, the detailed description of the invention of the above patent publication does not disclose that the extract has the effect of treating inflammation in respiratory diseases by inhibiting 5-lipoxygenase activity.

In addition, it was reported that a water extract of herbal plants, including Cnidii rhizoma, inhibited bronchospasm induced by histamine and acetylcholine in a guinea pig model and was effective against allergic infection, suggesting that it has the effect of preventing or treating asthma (Zhongguo Zhong Xi Yi Jie He Za Zhi, 1994, 14[8], 465-8). However, the extract is a water extract, and an alcohol extract is not disclosed in the above literature.

Further, Chinese Patent Publication No. 100348258 discloses that an herbal extract containing an extract of *Polygoni cuspidati radix* has therapeutic effects against tonsillitis, pharyngitis, laryngitis, parotitis and the like, but it relates to an herbal extract containing an extract of *Polygoni cuspidati radix* and does not disclose the effect of a mixture of Cnidii rhizoma and Polygoni cuspidati radix.

Korean Patent Registration No. 824970 discloses that an ethanol extract of *Polygoni cuspidati radix* has anti-allergic effects in diseases, including atopic dermatitis, seasonal allergy or asthma, but it does not disclose the effect of an alcohol extract of a mixture of Cnidii rhizoma and Polygoni cuspidati radix.

In addition, Korean Patent Publication No. 2010-4215 discloses that a water extract containing Cnidii rhizoma and Polygoni cuspidati radix and an ethyl acetate fraction thereof inhibited ear edema and the invasion of inflammatory cells in mice, suggesting that they have the effect of preventing or treating skin diseases by inhibiting inflammation. However, the herbal extract is a water or organic solvent extract, and the detailed description of Korean Patent Publication No. 2010-4215 does not disclose that the extract has 5-lipoxygenase inhibitory activity to exhibit airway constriction inhibitory activity, airway inflammation inhibitory activity, ear edema/inflammation inhibitory activity and antitussive and expectorant activities in respiratory diseases.

### Detailed Description of the Invention

### Technical Problem

It is an object of the present invention to provide a composition for preventing or treating respiratory diseases, which contains an herbal extract of a mixture of Cnidii rhizoma and Polygoni cuspidati radix as an active ingredient.

Another object of the present invention is to verify that an herbal extract of a mixture of Cnidii rhizoma and Polygoni cuspidati radix has significantly high 5-lipoxygenase inhibitory activity, airway constriction inhibitory activity, airway inflammation inhibitory activity, ear edema/inflammation inhibitory activity and antitussive and expectorant activities compared to an herbal extract of each of the plants.

### Technical Solution

The present invention is directed to a composition for preventing or treating respiratory diseases, which contains an herbal extract of a mixture of Cnidii rhizoma and Polygoni cuspidati radix, and a preparation method thereof.

As used herein, the term "extract" in the herbal extract of the mixture of Cnidii rhizoma and Polygoni cuspidati radix refers to a crude extract or a fraction which is a solvent extract of the crude extract. The extract may be in the form of solution, concentrate or dry powder.

In the present invention, it was found that the use of the herbal extract of the mixture of Cnidii rhizoma and Polygoni cuspidati radix shows significantly high 5-lipoxygenase inhibitory activity, airway constriction inhibitory activity, airway inflammation inhibitory activity, ear edema/inflammation inhibitory activity and antitussive and expectorant activities compared to the use of an herbal extract of each of Cnidii rhizoma and Polygoni cuspidati radix. Particularly, in an *in vivo* animal test, it was found that the herbal extract of the mixture has an excellent effect of treating respiratory diseases.

The weight ratio between Cnidii rhizoma and Polygoni cuspidati radix in the inventive herbal extract of the mixture of Cnidii rhizoma and Polygoni cuspidati radix is 0.1:1 to 10:1 (*Cnidii rhizoma* weight: *Polygoni cuspidati radix* weight), preferably 0.5:1 to 5:1, and more preferably 0.3:1 to 3:1.

The herbal extract of the mixture according to the present invention may be either a crude extract (primary extract) obtained by extracting the plants with a C₁-C₄ lower alcohol or an aqueous solution thereof, or a concentrate of the crude extract. Preferably, the C₁-C₄ lower alcohol may be selected from among ethanol, methanol, propanol, isopropanol, sec-butanol and butanol. The content of the C₁-C₄ lower alcohol in the aqueous solution may be 10-95% (v/v), and preferably 30-95% (v/v). When botanol is used as the extraction solvent, it is preferably water-saturated butanol (75-85% butanol aqueous solution). The extraction temperature is 40∼120 °C, and preferably 60∼90 °C, and the extraction time is 2-24 hours, and preferably 4-12 hours.

In addition, the crude extract (primary extract) of the mixture of Cnidii rhizoma and Polygoni cuspidati radix may be concentrated, and the concentrate may be fractionated with a C₁-C₄ lower alcohol, an aqueous solution thereof, a non-polar solvent, or a combination thereof, thereby obtaining a fraction (secondary extract). The C₁-C₄ lower alcohol may be selected from among ethanol, methanol, propanol, isopropanol and butanol. The non-polar solvent may be selected from the group consisting of ethyl acetate, dichloromethane, chloroform, and butanol. Herein, the extraction temperature is 40∼120 °C, and preferably 60∼90 °C, and the extraction time is 2-24 hours, and preferably 4-12 hours.

In addition, a fraction (secondary extract) may be obtained by suspending the concentrate of the crude extract (primary extract) of the mixture of Cnidii rhizoma and Polygoni cuspidati radix in a 5-20-fold volume (v/w) of purified water and extracting the suspension with one or more solvents selected from the group consisting of ethyl acetate, dichloromethane, chloroform and butanol (including water-saturated butanol). Herein, the extraction temperature is 40∼120 °C, and preferably 60∼90 °C, and the extraction time is 2-24 hours, and preferably 4-12 hours. In the present invention, the number of extractions is not limited and is determined according to the efficiency of extraction. If the amount of solvent that is used in the preparation of the extract of the mixture of Cnidii rhizoma and Polygoni cuspidati radix is too small, the extraction efficiency will be reduced due to the low solubility of the extract, and a filter can be plugged during filtration, and if the amount of the solvent is excessively large, an increase in the amount of lower alcohol used can lead to a decrease in economic efficiency and cause problems associated with handling. For this reason, the amount of solvent used is preferably in the above-described range.

In the present invention, the method of fractionating the primary extract is selected in order to prevent the extraction efficiency from being reduced due to the loss of a large amount of the extract, caused by a large amount of the extraction solvent, even when the herbal plants are extracted in large amounts and the primary extract is filtered. In addition, the extraction efficiency in each extraction step was examined, and as a result, it was found that the amount of extraction in the first and second extraction steps was about 90% of the total amount of extraction and that a method comprising three or more extraction steps was not efficient, because economic efficiency versus yield was low.

In order to remove the remaining lower alcohol from the crude extract or fraction obtained as described above so as to make the crude extract or the fraction suitable for use as a medical raw material, the crude extract or the fraction may be azeotropically concentrated 2-8 times with water in an amount of 2-10 times (v/w), and preferably 2-4 times (v/w), the weight of the extract. The concentrate may be suspended uniformly in water, and then dried using a conventional drying method such as vacuum drying, spray drying or freeze drying, thereby obtaining a mixed extract of Cnidii rhizoma and Polygoni cuspidati radix in a powder state.

The extraction method that is used in the present invention may be any conventional extraction method. For example, the extraction method may be a dipping (cold or hot extraction), hot-water extraction, ultrasonic extraction or reflux cooling extraction method, but is not limited thereto.

In another aspect of the present invention, the extract of the mixture of Cnidii rhizoma and Polygoni cuspidati radix shows significantly high 5-liposygenase inhibitory activity, airway constriction inhibitory activity, airway inflammation inhibitory activity, ear edema/inflammation inhibitory activity and antitussive and expectorant activities, compared to an herbal extract of each of the plants, and thus can be effectively used for the prevention or treatment of respiratory diseases or the relief of respiratory disease symptoms.

Thus, the present invention provides a composition for preventing or treating respiratory diseases, which contains the herbal extract of mixture of Cnidii rhizoma and Polygoni cuspidati radix as an active ingredient.

The respiratory disease may be any disease that causes inflammation and airway constriction. Generally, it may be a disease selected from the group consisting of acute and chronic bronchitis, catarrhal bronchitis, obstructive bronchitis, inflammatory bronchitis, bronchial asthma, atopic asthma, non-atopic asthma, atopic IgE-mediated asthma, allergic asthma, non-allergic asthma, chronic bronchial constriction, acute bronchial constriction, chronic obstructive pulmonary disease, coughing, phlegm, bronchial adenoma, pulmonary tuberculosis, pulmonary emphysema and lung abscess.

The content of the mixed extract as an active ingredient in the inventive composition can be suitably determined depending on the dosage form and intended use of the composition, the patient's severity, etc., may be 0.01-99.9 wt% (on a solid weight basis), and preferably 0.1-50 wt%, but is not limited thereto.

In addition, the composition of the present invention may be administered as various oral and parenteral formulations in actual clinical applications. The composition of the present invention may be formulated with commonly used diluents or excipients, such as fillers, extenders, binders, wetting agents, disintegrants, surfactants, etc. Solid formulations for oral administration include tablets, pills, powders, granules, and capsules, and such solid formulations can be prepared by mixing either a crude extract of a mixture of Cnidii rhizoma and Polygoni cuspidati radix, a solvent extract of the crude extract, or a compound isolated therefrom, with one or more excipients, for example, starch, cellactose, calcium carbonate, Crospovidone, light anhydrous silicic acid, lactose, stearic acid, magnesium, talc, enzymatically treated stevia, microcrystalline cellulose, magnesium stearate, or gelatin. Liquid formulations for oral administration include suspensions, solutions, emulsions, and syrup, and may contain various excipients, for example, wetting agents, flavoring agents, aromatics and preservatives, in addition to water and liquid paraffin, which are frequently used simple diluents. Formulations for parenteral administration include sterilized aqueous solutions, non-aqueous solutions, suspensions, emulsions, and freeze-dried preparations. As non-aqueous solvents or suspending agents, propylene glycol, polyethylene glycol, plant oils such as olive oil, injectable esters such as ethyl oleate, and the like can be used.

The dosage of the inventive composition containing the mixed extract may vary depending on the patient's weight, age, sex, physical condition and diet, the time of administration, the mode of administration, excretion rate, and the severity of the disease. The inventive composition may be administered once or several times a day at predetermined time intervals according to the physician's or pharmacist's judgment. For example, the active ingredient of the inventive composition may be administered at a daily dose of 0.001-500 mg/kg, and preferably 0.1-200 mg/kg.

In another aspect, the present invention provides a health functional food for preventing or alleviating respiratory diseases, including asthma, acute bronchitis, and chronic obstructive pulmonary disease, which contains the extract of the mixture of Cnidii rhizoma and Polygoni cuspidati radix. The health functional food may be selected from various foods, beverages, food additives and the like.

The content of the active ingredient extract in the health functional food can be suitably determined depending on the type of food and the intended use. For example, the content of the extract may be 0.01-50 wt%, and preferably 0.1-10 wt%, based on the total weight of the food. In addition, the content of the extract in the health beverage composition may be 0.02-10 g, and preferably 0.1-5 g, based on 100 ml of the composition.

Providing that the health beverage composition of the present invention contains the above-described extract as an essential component in the indicated ratio, there is no particular limitation on liquid components, and the composition may further contain various flavoring agents or natural carbohydrates, like conventional beverages. Examples of the above-described natural carbohydrate include monosaccharides such as glucose or fructose, disaccharides such as maltose or sucrose, and polysaccharides, for example, conventional sugars such as dextrin or cyclodextrin, and sugar alcohols such as xylitol or erythritol. In addition, examples of flavoring agents that may be used in the present invention include natural flavoring agents (taumatin, stevia extracts such as levaudioside A, glycyrrhizin, etc.) and synthetic flavoring agents (saccharin, aspartame, etc.). The content of the natural carbohydrate in the composition of the present invention is generally about 1-20 g, and preferably 5-12 g, based on 100 ml of the composition.

In addition, the composition of the present invention may contain various nutrients, vitamins, minerals (electrolytes), flavoring agents such as synthetic flavoring agents and natural flavoring agents, coloring agents, fillers (cheese, chocolate, etc.), pectic acid and its salt, alginic acid and its salt, organic acid, a protective colloidal thickener, a pH controlling agent, a stabilizer, a preservative, glycerin, alcohol, a carbonizing agent used in carbonic acid beverages, etc. In addition, the composition of the present invention may contain flesh for preparing natural fruit juice, fruit juice beverages and vegetable beverages. Such components may be used alone or in combination. The content of such additives is not so critical to the present invention, but is generally selected in the range of about 0.001-20 parts by weight based on 100 parts by weight of the composition of the present invention.

Particularly, Cnidii rhizoma and Polygoni cuspidati radix are herbal materials which have been used in Chinese medicine, and it is believed that, when these herbal materials are administered to the human body, they will have fewer side effects compared to other synthetic medical drugs. The toxicity of the standardized composition containing the herbal extract was tested in an animal test, and as a result, it was identified that the composition was not toxic *in vivo*.

### Advantageous Effects

The inventive extract of the mixture of Cnidii rhizoma and Polygoni cuspidati radix has excellent 5-liposygenase inhibitory activity, airway constriction inhibitory activity, airway inflammation inhibitory activity, ear edema/inflammation inhibitory activity and antitussive and expectorant activities, and such activities are significantly higher in the mixed extract (extract of Cnidii rhizoma and Polygoni cuspidati radix mixed at the optimum ratio) or a fraction thereof than in an extract of each of Cnidii rhizoma and Polygoni cuspidati radix. In addition, the composition of the present invention shows good effects in various antiasthma disease animal models, unlike conventional therapeutic agents, and thus will be highly useful against respiratory diseases, including asthma (bronchial asthma, atopic asthma, non-atopic asthma, atopic IgE-mediated asthma, allergic asthma and non-allergic asthma), acute and chronic bronchitis, chronic obstructive pulmonary disease, bronchial adenoma, pulmonary tuberculosis, pyothrox, lung abscess, coughing, phlegm, allergic rhinitis, acute lower respiratory infections (bronchitis and bronchiolitis), catarrhal bronchitis, obstructive or inflammatory bronchial disease, laryngopharyngitis, tonsillitis and laryngitis.

### Brief Description of the Drawings

FIG. 1 shows bronchoconstriction inhibitory activity after treating the extracted bronchi of guinea pigs with 0.3 mg/mℓ of each of extracts of Examples 1 to 44 and Comparative Examples 1 to 5.
FIG. 2 shows the results of analysis conducted to examine whether an extract of Example 2 inhibits the expression of co-stimulatory molecules in the dendritic cells of the lung tissue of an ovalbumin-induced asthma/chronic obstructive pulmonary disease model.
FIG. 3 shows the results of analysis conducted to examine whether an extract of Example 2 inhibits the invasion of inflammatory cells into the lung tissue of an ovalbumin-induced asthma/chronic obstructive pulmonary disease model.
FIG. 4 shows the results of analysis conducted to examine whether an extract of Example 2 inhibits the expression of co-stimulatory molecules in the dendritic cells of the lung tissue of an ovalbumin- and LPS-induced asthma/chronic obstructive pulmonary disease model.
FIG. 5 shows the results of analysis conducted to examine whether an extract of Example 2 inhibits the expression of co-stimulatory molecules in the dendritic cells of the lung tissue of an *E. coli*-EV-induced asthma/chronic obstructive pulmonary disease model.

### Mode for Carrying Out the Invention

Hereinafter, preferred examples of the present invention will be described in detail. However, the present invention is not limited to the examples described herein and can be embodied in various forms. The examples disclosed herein are provided so that the disclosure becomes thorough and complete and the spirit of the present invention is sufficiently delivered to those skilled in the art.

### Example 1: Preparation of 50% ethanol aqueous solution extract of Cnidii rhizoma + Polygoni cuspidati radix (1:1)

50 g of *Cnidii rhizoma* and 50 g of *Polygoni cuspidati radix* were mixed with each other, and the mixture was extracted twice with 1000 mℓ of 50% ethanol aqueous solution under reflux cooling at 88 °C for 4 hours each time. The extract was filtered and concentrated under reduced pressure. The obtained concentrate was suspended in a 5-fold weight of distilled water, and then freeze-dried, thereby obtaining 29.8 g of an extract of a mixture of Cnidii rhizoma and Polygoni cuspidati radix.

### Example 1: Preparation of butanol fraction of 50% ethanol aqueous solution extract of Cnidii rhizoma + Polygoni cuspidati radix (1:1)

The crude extract obtained in Example 1 was suspended in a 10-fold volume (v/w) of purified water, and the suspension was added to the same amount of water-saturated butanol (80% butanol aqueous solution) so as to be separated into layers. The layer separation was performed twice. Then, the butanol layer was collected, filtered and concentrated under reduced pressure. After evaporation of butanol and purified water, the residue was azeotropically concentrated in about 100 mℓ of purified water, and the azeotropic concentration was performed twice, thereby completely removing the butanol. The resulting concentrate was suspended in a five-fold weight of distilled water, and then freeze-dried, thereby obtaining 5.6 g of a fraction as powder.

Examples 3 to 44 and Comparative Examples 1 to 5 were performed in the same manner as described in Examples 1 and 2 under the conditions shown in Table 1 below. However, a water extract was obtained by extracting plants with water under reflux cooling for 8 hours, filtering the extract and immediately freeze-drying the filtrate.

**Table 1**

| Conditions | Herbal extracts | | | | Concentration production (%) |
|---|---|---|---|---|---|
| | *CNIDII RHIZO MA*(g) | *POLYGONI CUSPIDATI* RADIX(g) | Solvent for preparation of crude extract | Solvent for preparation of fraction | |
| Example 3 | 50 | 50 | 50% ethanol aqueous solution | Ethyl acetate | 3.7 |
| Example4 | 50 | 50 | 50% ethanol aqueous solution | Dichloromethane | 2.1 |
| Example 5 | 50 | 50 | 10% ethanol aqueous solution | - | 22.3 |
| Example 6 | 50 | 50 | 10% ethanol aqueous solution | Water saturated butanol | 3.9 |
| Example 7 | 50 | 50 | 30% ethanol aqueous solution | - | 23.5 |
| Example 8 | 50 | 50 | 30% ethanol aqueous solution | Water saturated butanol | 4.1 |
| Example 9 | 50 | 50 | 70% ethanol aqueous solution | - | 28.7 |
| Example 10 | 50 | 50 | 70% ethanol aqueous solution | Water saturated butanol | 6.2 |
| Example 11 | 50 | 50 | 70% ethanol aqueous solution | Ethyl acetate | 3.9 |
| Example 12 | 50 | 50 | 70% ethanol aqueous solution | Dichloromethane | 2.4 |
| Example 13 | 50 | 50 | 95% ethanol aqueous solution | - | 12.8 |
| Example 14 | 50 | 50 | 95% ethanol aqueous solution | Water saturated butanol | 3.2 |
| Example 15 | 50 | 50 | Water saturated butanol | - | 11.7 |
| Example 16 | 50 | 50 | 30% methanol aqueous solution | - | 25.4 |
| Example 17 | 50 | 50 | 30% methanol aqueous solution | Water saturated butanol | 4.8 |
| Example 18 | 50 | 50 | 50% methanol aqueous solution | - | 32.1 |
| Example 19 | 50 | 50 | 50% methanol aqueous solution | Water saturated butanol | 6.7 |
| Example 20 | 50 | 50 | 70% methanol aqueous solution | - | 27.5 |
| Example 21 | 50 | 50 | 70% methanol aqueous solution | Water saturated butanol | 8.1 |
| Example 22 | 50 | 50 | 50% isopropanol aqueous solution | - | 23.3 |
| Example 23 | 50 | 50 | 50% isopropanol aqueous solution | Water saturated butanol | 4.7 |
| Example 24 | 50 | 50 | 50% propanol aqueous solution | - | 21.2 |
| Example 25 | 66 | 34 | 50% propanol aqueous solution | Water saturated butanol | 4.4 |
| Example 26 | 66 | 34 | 50% ethanol aqueous solution | - | 22.6 |
| Example 27 | 66 | 34 | 50% ethanol aqueous solution | Water saturated butanol | 4.5 |
| Example 28 | 66 | 34 | 50% methanol aqueous solution | - | 29.4 |
| Example 29 | 66 | 34 | 50% methanol aqueous solution | Water saturated butanol | 5.8 |
| Example 30 | 66 | 34 | Water saturated butanol | - | 9.8 |
| Example 31 | 66 | 34 | 50% isopropanol aqueous solution | - | 22.1 |
| Example 32 | 66 | 34 | 50% isopropanol aqueous solution | Water saturated butanol | 5.1 |
| Example 33 | 66 | 34 | 50% propanol aqueous solution | - | 20.4 |
| Example 34 | 66 | 34 | 50% propanol aqueous solution | Water saturated butanol | 4.7 |
| Example 35 | 80 | 20 | 50% ethanol aqueous solution | - | 25.2 |
| Example 36 | 80 | 20 | 50% ethanol aqueous solution | Water saturated butanol | 3.6 |
| Example 37 | 80 | 20 | Water saturated butanol | - | 6.8 |
| Example 38 | 34 | 66 | 50% ethanol aqueous solution | - | 33.2 |
| Example 39 | 34 | 66 | 50% ethanol aqueous solution | Water saturated butanol | 6.3 |
| Example 40 | 34 | 66 | Water saturated butanol | - | 14.2 |
| Example 41 | 20 | 80 | 50% ethanol aqueous solution | - | 27.1 |
| Example 42 | 20 | 80 | 50% ethanol aqueous solution | Water saturated butanol | 6.6 |
| Example 43 | 20 | 80 | Water saturated butanol | - | 12.7 |
| Example 44 | 50 | 50 | 100% ethanol | - | 7.9 |
| Comparativ e Example 1 | 100 | - | 50% ethanol aqueous solution | - | 23.4 |
| Comparativ e Example 2 | - | 100 | 50% ethanol aqueous solution | - | 32.2 |
| Comparativ e Example 3 | 100 | - | Water | - | 18.7 |
| Comparativ e Example 4 | - | 100 | Water | - | 22.3 |
| Comparativ e Example 5 | 50 | 50 | Water | - | 20.2 |

### Test Example 1: 5-lipoxygenase inhibitory activity

To examine whether the mixed extracts prepared in Examples 1 to 44 and the extracts prepared in Comparative Examples 1 to 5 have an inhibitory effect against 5-lipoxygenase (leukotriene synthase) which is the major cause of asthma, the following test was performed.

Specifically, rat basophilic leukemia cells (RBL-1) were stabilized using serum-free RPMI medium at 37 °C, and each of the extracts and arachidonic acid as a substrate were added thereto and incubated for 15 minutes to produce leukotriene. The amount of the produced leukotriene was measured using a cysteinyl leukotriene EIA kit. As comparative drugs, the 5-lipoxygenase inhibitors zileuton and montelukast were used.

**Table 2**

| Conditions | | Concentration (*µ*g/ml) | 5-lipoxygenase inhibition rate (%) |
|---|---|---|---|
| Example 1 | | 10 | 52 |
| | | 50 | 90 |
| Example 2 | | 10 | 79 |
| | | 50 | 98 |
| Example 3 | | 10 | 56 |
| | | 50 | 91 |
| Example 4 | | 10 | 42 |
| | | 50 | 82 |
| Example 6 | | 10 | 44 |
| | | 50 | 81 |
| Example 7 | | 10 | 46 |
| | | 50 | 84 |
| Example 8 | | 10 | 51 |
| | | 50 | 92 |
| Example 9 | | 10 | 48 |
| | | 50 | 87 |
| Example 10 | | 10 | 63 |
| | | 50 | 92 |
| Example 11 | | 10 | 54 |
| | | 50 | 96 |
| Example 12 | | 10 | 56 |
| | | 50 | 78 |
| Example 14 | | 10 | 56 |
| | | 50 | 92 |
| Example 15 | | 10 | 61 |
| | | 50 | 90 |
| Example 16 | | 10 | 36 |
| | | 50 | 77 |
| Example 17 | | 10 | 43 |
| | | 50 | 80 |
| Example 18 | | 10 | 39 |
| | | 50 | 75 |
| Example 19 | | 10 | 55 |
| | | 50 | 94 |
| Example 20 | | 10 | 41 |
| | | 50 | 76 |
| Example 21 | | 10 | 51 |
| | | 50 | 88 |
| Example 23 | | 10 | 59 |
| | | 50 | 93 |
| Example 25 | | 10 | 71 |
| | | 50 | 94 |
| Example 27 | | 10 | 68 |
| | | 50 | 89 |
| Example 29 | | 10 | 57 |
| | | 50 | 89 |
| Example 30 | | 10 | 35 |
| | | 50 | 74 |
| Example 32 | | 10 | 68 |
| | | 50 | 91 |
| Example 34 | | 10 | 49 |
| | | 50 | 83 |
| Example 36 | | 10 | 38 |
| | | 50 | 83 |
| Example 39 | | 10 | 63 |
| | | 50 | 90 |
| Example 40 | | 10 | 47 |
| | | 50 | 81 |
| Example 42 | | 10 | 36 |
| | | 50 | 79 |
| Example 44 | | 10 | 44 |
| | | 50 | 82 |
| Comparative Example 1 | | 10 | 8 |
| | | 50 | 27 |
| Comparative Example 2 | | 10 | 17 |
| | | 50 | 33 |
| Comparative Example 3 | | 10 | 9 |
| | | 50 | 24 |
| Comparative Example 4 | | 10 | 13 |
| | | 50 | 20 |
| Comparative Example 5 | | 10 | 10 |
| | | 50 | 21 |
| Comparative drugs | Zileuton | 1 µM | 63 |
| | montelukast | 2 µM | 45 |

As can be seen in Table 2 above, the mixed extracts of Examples 1 to 44 of the present invention had excellent inhibitory effects against 5-liposygernase, and the extract of the mixture of Cnidii rhizoma and Polygoni cuspidati radix or the fraction thereof had a significant inhibitory effect against 5-lipoxygenase due to the synergistic effect of the two plants compared to the extract of each of the plants.

### Test Example 2: Test for bronchoconstriction (in vitro)

In order to examine whether 18 mixed extracts which showed the highest inhibitory activity against 5-lipoxygenase in Test Example 1 and the extracts of Comparative Examples 1 to 5 have bronchoconstriction inhibitory activity, the following test was performed (see Nature, 1977, 270, 256-257; Jpn. J. Pharmacol., 1996, 72, 1-8; Kor. J. Pharmacogn. 1999, 30[4], 377-383). The comparative drug rolipram was used at a concentration of 20 µM, and each of the extracts was used at a concentration of 0.3 mg/mℓ.

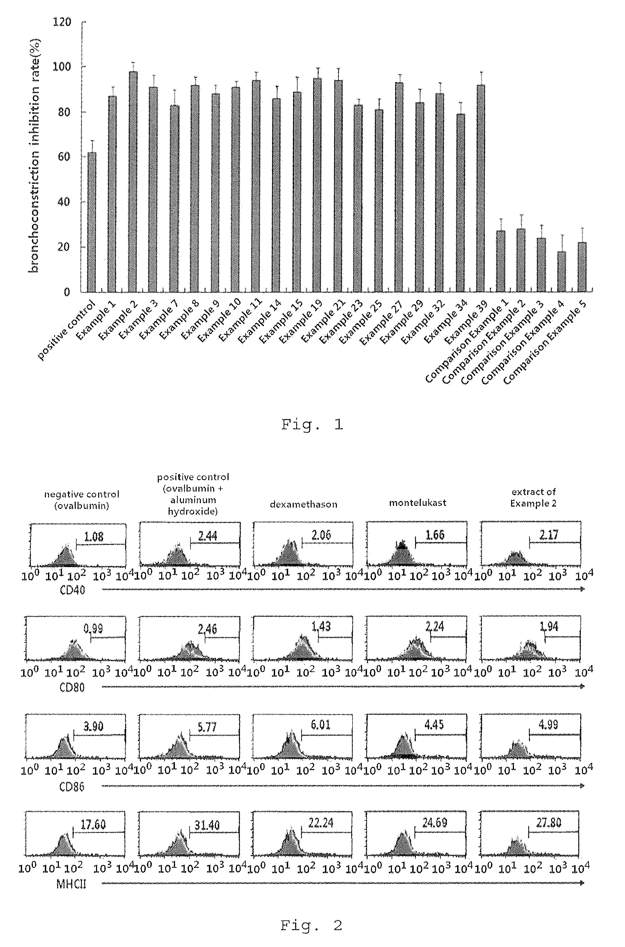

Specifically, Hartely male guinea pigs (400-500 g, BGI, Korea) were sensitized by intravenous injection with 2 mℓ/kg of anti-ovalbumin anti-serum. At 48 hours after sensitization, the guinea pigs were sacrificed and the bronchi were extracted therefrom. Then, other tissues attached to the bronchi were removed using a tyrode solution, and each of the bronchi was cut in a ring shape containing 2-3 cartilages. Then, the cartilage portion of the ring was excised while the bronchial muscle was preserved. Then, a string was connected to both sides of each bronchus which was then suspended in an organ bath and stabilized for a predetermined time. Then, 10 *µ*g/mℓ of carbachol was added to induce the maximum constriction of the bronchi which were then washed with a tyrode solution and stabilized. Next, 5 µM of indomethacin was added to each bronchus, and after 1 minute, each of the extracts was added to each bronchi, and after 5 minutes, 20 *µ*g/mℓ of ovalbumin was added to induce bronchoconstriction. The inhibition rate(%) of bronchoconstriction was calculated by comparing the constrictions caused by carbachol and ovalbumin, and the results of the calculation are shown in FIG. 1 and Table 3 below. The relaxation of the bronchi was measured using a physiological activity meter connected (powerlab 8/30, AD Instument) with a force transducer (WPI).

**Table 3**

| Conditions | | Concentration (mg/ml) | Bronchoconstriction inhibition rate(%) |
|---|---|---|---|
| Example 1 | | 0.03 | 19 |
| | | 0.1 | 39 |
| | | 0.3 | 87 |
| Example 2 | | 0.03 | 31 |
| | | 0.1 | 64 |
| | | 0.3 | 98 |
| Example 3 | | 0.03 | 28 |
| | | 0.1 | 57 |
| | | 0.3 | 91 |
| Example 8 | | 0.03 | 28 |
| | | 0.1 | 52 |
| | | 0.3 | 92 |
| Example 9 | | 0.03 | 23 |
| | | 0.1 | 48 |
| | | 0.3 | 88 |
| Example 10 | | 0.03 | 31 |
| | | 0.1 | 62 |
| | | 0.3 | 91 |
| Example 11 | | 0.03 | 25 |
| | | 0.1 | 47 |
| | | 0.3 | 94 |
| Example 15 | | 0.03 | 19 |
| | | 0.1 | 46 |
| | | 0.3 | 89 |
| Example 19 | | 0.03 | 27 |
| | | 0.1 | 63 |
| | | 0.3 | 95 |
| Example 21 | | 0.03 | 24 |
| | | 0.1 | 48 |
| | | 0.3 | 94 |
| Example 27 | | 0.03 | 20 |
| | | 0.1 | 51 |
| | | 0.3 | 93 |
| Example 32 | | 0.03 | 24 |
| | | 0.1 | 48 |
| | | 0.3 | 88 |
| Example 39 | | 0.03 | 29 |
| | | 0.1 | 56 |
| | | 0.3 | 92 |
| Comparative Example 1 | | 0.03 | 8 |
| | | 0.1 | 18 |
| | | 0.3 | 27 |
| Comparative Example 2 | | 0.03 | 9 |
| | | 0.1 | 16 |
| | | 0.3 | 28 |
| Comparative Example 3 | | 0.03 | 6 |
| | | 0.1 | 13 |
| | | 0.3 | 24 |
| Comparative Example 4 | | 0.03 | 4 |
| | | 0.1 | 13 |
| | | 0.3 | 18 |
| Comparative Example 5 | | 0.03 | 5 |
| | | 0.1 | 14 |
| | | 0.3 | 22 |
| Comparative drug | rolipram | 20 µM | 62 |

As can be seen in FIG. 1 and Table 3 above, the mixed extracts of Cnidii rhizoma and Polygoni cuspidati radix of the Examples showed excellent bronchoconstriction inhibitory activity in a dose-dependent manner. In addition, the mixed extract of Cnidii rhizoma and Polygoni cuspidati radix or the fraction thereof had a significant inhibitory effect compared to the extract of each of the plants.

### Test Example 3: Test for airway constriction inhibition (in vivo)

In order to examine whether 13 extracts of the Examples which showed excellent bronchoconstriction inhibitory activity in Test Example 2 and the extracts of Comparative Examples 1 to 5 have airway constriction inhibitory activity, the following test was performed (test method - measurement of breathing: European Journal of Pharmacology, 2000, 403, 169-179; Br. J. Pharmac., 1983, 78, 67-74).

Specifically, Hartely male guinea pigs (400-450 g, SLC, Japan) were sensitized by intravenous injection with 1.5 mℓ/kg of anti-ovalbumin anti-serum. At 48 hours after sensitization, the test materials were administered orally to the guinea pigs. At 30 minutes after oral administration, the guinea pigs were pretreated by subcutaneous injection with pyrilamine maleate (0.5 mg/kg) and propranolol (0.05 mg/kg), and in order to measure various breathing indices, the guinea pigs were placed in plethysmograph box type 855, HSE, Germany) equipped with a plethysmometer, and basal airway resistance (RxV) was measured. At 30 minutes after the pretreatment, aerosol prepared from 1% ovalbumin using high-pressure compressed air was sprayed into the chamber for 2 minutes, and then albumin-containing air was discharged from the chamber for 30 seconds. Then, the airway resistance in the plethysmograph box was measured for 15 minutes, and the airway resistance was calculated as AUC (area under the curve) and shown as airway constriction inhibition rate(%) in comparison with a comparative drug in Table 4 below. As a comparative drug, montelukast was used.

**Table 4**

| Conditions | | Oral dose (mg/kg) | Airway constriction inhibition rate (%) |
|---|---|---|---|
| Example 1 | | 100 | 19 |
| | | 200 | 31 |
| | | 400 | 51 |
| Example 2 | | 100 | 41 |
| | | 200 | 47 |
| | | 400 | 63 |
| Example 3 | | 100 | 15 |
| | | 200 | 32 |
| | | 400 | 54 |
| Example 8 | | 100 | 27 |
| | | 200 | 38 |
| | | 400 | 65 |
| Example 9 | | 100 | 16 |
| | | 200 | 32 |
| | | 400 | 47 |
| Example 10 | | 100 | 24 |
| | | 200 | 33 |
| | | 400 | 52 |
| Example 11 | | 100 | 26 |
| | | 200 | 37 |
| | | 400 | 65 |
| Example 15 | | 100 | 18 |
| | | 200 | 30 |
| | | 400 | 46 |
| Example 19 | | 100 | 22 |
| | | 200 | 30 |
| | | 400 | 51 |
| Example 21 | | 100 | 23 |
| | | 200 | 31 |
| | | 400 | 55 |
| Example 27 | | 100 | 22 |
| | | 200 | 33 |
| | | 400 | 54 |
| Example 32 | | 100 | 21 |
| | | 200 | 32 |
| | | 400 | 51 |
| Example 39 | | 100 | 22 |
| | | 200 | 35 |
| | | 400 | 55 |
| Comparative Example 1 | | 200 | 9 |
| | | 400 | 18 |
| Comparative Example 2 | | 200 | 11 |
| | | 400 | 18 |
| Comparative Example 3 | | 200 | 7 |
| | | 400 | 17 |
| Comparative Example 4 | | 200 | 10 |
| | | 400 | 17 |
| Comparative Example 5 | | 200 | 9 |
| | | 400 | 16 |
| Comparative drug | montelukast | 10 | 53 |

As can be seen in Table 4 above, the mixed extracts of Cnidii rhizoma and Polygoni cuspidati radix of the examples of the present invention showed excellent inhibitory effects against the airway constriction of the sensitized guinea pigs in a dose-dependent manner. In addition, the mixed extract of Cnidii rhizoma and Polygoni cuspidati radix or the fraction thereof showed a significant inhibitory effect against airway constriction compared to the extract of each of the plants.

### Test Example 4: Test for inhibition of bronchial inflammation

In order to examine whether 13 extracts of the Examples which showed excellent bronchoconstriction inhibitory activity in Test Example 2 and the extracts of Comparative Examples 1 to 5 have an inhibitory effect against pulmonary bronchial inflammation in an asthma/chronic obstructive pulmonary disease model, an increase in leukocytes of the pulmonary bronchus, caused by exposure of sensitized mice to antigen, was examined (Pharmacological Research, 2010, 61, 288-297; Biochemical Pharmacology, 2010, 79, 888-896).

Specifically, BALB/c female mice (6.5 weeks old, SLC, Japan) were sensitized by intraperitoneal administration of 0.2 mℓ of a 1:1 mixture of 10 *µ*g of ovalbumin (OVA, Sigma) and 4 mg of aluminum hydroxide (Alum, Pierce) at days 0, 7 and 14. At 8 days and 10 days after the final sensitization, aerosol prepared from 1.0% ovalbumin using high-pressure compressed air was sprayed into the mice for 50 minutes to induce airway inflammation. As a comparative drug, 10 mg/kg of rolipram (Sigma) was used. The comparative drug and the test materials were orally administered twice (morning and evening) a day during a period ranging from 21 days to 23 days after the first sensitization. At 24 hours after the final induction of inflammation, the bronchoalveoli were laved with 1.5 mℓ of phosphate buffered saline (pH 7.2) to collect a bronchoalveolar lavage. The number of leukocytes in the lavage was counted using a hematology analyzer (Drew Scientific Inc., HEMAVET HV950FS, M-950HV). Based on the results of the measurement, the inhibition rate (%) of pulmonary bronchial inflammation was calculated relative to the number of leukocytes in a negative control (administered with 1% CMC (carboxymethyl cellulose)), and the results of the calculation are shown in Table 5 below.

**Table 5**

| Conditions | | Oral dose (mg/kg, *b.i.d.*) | Inhibition rate (%) of pulmonary bronchial inflammation |
|---|---|---|---|
| Example 1 | | 100 | 13 |
| | | 200 | 19 |
| | | 400 | 26 |
| Example 2 | | 100 | 18 |
| | | 200 | 26 |
| | | 400 | 39 |
| Example 3 | | 100 | 15 |
| | | 200 | 32 |
| | | 400 | 34 |
| Example 8 | | 100 | 15 |
| | | 200 | 23 |
| | | 400 | 35 |
| Example 9 | | 100 | 13 |
| | | 200 | 18 |
| | | 400 | 24 |
| Example 10 | | 100 | 14 |
| | | 200 | 22 |
| | | 400 | 34 |
| Example 11 | | 100 | 26 |
| | | 200 | 37 |
| | | 400 | 65 |
| Example 15 | | 100 | 11 |
| | | 200 | 17 |
| | | 400 | 24 |
| Example 19 | | 100 | 16 |
| | | 200 | 23 |
| | | 400 | 33 |
| Example 21 | | 100 | 18 |
| | | 200 | 24 |
| | | 400 | 35 |
| Example 27 | | 100 | 14 |
| | | 200 | 23 |
| | | 400 | 37 |
| Example 32 | | 100 | 11 |
| | | 200 | 22 |
| | | 400 | 33 |
| Example 39 | | 100 | 15 |
| | | 200 | 25 |
| | | 400 | 37 |
| Comparative Example 1 | | 100 | 4 |
| | | 200 | 9 |
| | | 400 | 11 |
| Comparative Example 2 | | 100 | 3 |
| | | 200 | 7 |
| | | 400 | 12 |
| Comparative Example 3 | | 100 | 2 |
| | | 200 | 5 |
| | | 400 | 8 |
| Comparative Example 4 | | 100 | 2 |
| | | 200 | 7 |
| | | 400 | 10 |
| Comparative Example 5 | | 100 | 3 |
| | | 200 | 9 |
| | | 400 | 12 |
| Comparative drug | rolipram | 10 | 22 |

As can be seen in Table 5 above, the extracts of the present invention inhibited the number of leukocytes in the bronchoalveolar lavage in a dose-dependent manner, suggesting that the extracts have excellent inflammation inhibitory activity. In addition, the mixed extract of Cnidii rhizoma and Polygoni cuspidati radix or the fraction thereof showed significantly high inhibitory activity against inflammation compared to the extract of each of the plants.

### Test Example 5: Test for anti-inflammatory effect against ear welling

In order to examine whether 13 extracts of the Examples which showed excellent bronchoconstriction inhibitory activity in Test Example 2 and the extracts of Comparative Examples 1 to 5 have anti-inflammatory activity, mice with edema induced by croton oil were used. When croton oil is applied to the skin, an inflammatory reaction occurs to produce redness, a swelling and a blister on the applied surface (Archives of Pharmacal Research, 1993, 16[1], 18-24).

As experimental animals, male ICR mice (weighed 20-23 g; Orientbio, Korea) were divided into groups, each consisting of 10 mice. Each of the test samples and the comparative drug COX-2 inhibitor Celecoxib (100 mg/kg, Pfizer) was orally administered into the mice which have been fasted for one day, and after 1 hour, a solution of 3% croton oil in acetone was applied uniformly to the inner and outer sides of the right ear of each mouse to induce ear edema. At 4 hours after induction of the ear edema, the mice were over-anesthetized with ether, and then the degree of ear edema of each mouse was measured by a rate change method using a thickness gauge, and the inhibition rate (%) was calculated relative to the degree of edema of a negative control group (administered with 1% CMC), and the results of the calculation are shown in Table 6 below.

**Table 6**

| Conditions | | Oral dose (mg/kg) | Ear edema inhibition rate (%) |
|---|---|---|---|
| Example 1 | | 100 | 13 |
| | | 200 | 24 |
| | | 400 | 34 |
| Example 2 | | 100 | 20 |
| | | 200 | 31 |
| | | 400 | 42 |
| Example 3 | | 100 | 11 |
| | | 200 | 22 |
| | | 400 | 33 |
| Example 8 | | 100 | 14 |
| | | 200 | 21 |
| | | 400 | 36 |
| Example 9 | | 100 | 10 |
| | | 200 | 19 |
| | | 400 | 25 |
| Example 10 | | 100 | 12 |
| | | 200 | 23 |
| | | 400 | 38 |
| Example 11 | | 100 | 11 |
| | | 200 | 27 |
| | | 400 | 35 |
| Example 15 | | 100 | 9 |
| | | 200 | 19 |
| | | 400 | 26 |
| Example 19 | | 100 | 12 |
| | | 200 | 27 |
| | | 400 | 35 |
| Example 21 | | 100 | 13 |
| | | 200 | 22 |
| | | 400 | 36 |
| Example 27 | | 100 | 16 |
| | | 200 | 27 |
| | | 400 | 35 |
| Example 32 | | 100 | 12 |
| | | 200 | 22 |
| | | 400 | 34 |
| Example 39 | | 100 | 15 |
| | | 200 | 24 |
| | | 400 | 31 |
| Comparative Example 1 | | 200 | 5 |
| | | 400 | 8 |
| Comparative Example 2 | | 200 | 5 |
| | | 400 | 7 |
| Comparative Example 3 | | 200 | 2 |
| | | 400 | 5 |
| Comparative Example 4 | | 200 | 5 |
| | | 400 | 8 |
| Comparative Example 5 | | 200 | 4 |
| | | 400 | 9 |
| Comparative drug | Celecoxib | 100 | 17 |

As can be seen in Table 6 above, the extract of the present invention showed excellent inhibitory activity against inflammation in the croton oil-induced ear edema animal model in a dose-dependent manner. In addition, the mixed extract of Cnidii rhizoma and Polygoni cuspidati radix or the fraction thereof showed a significantly high inhibitory effect compared to the extract of each of the plants.

### Test Example 6: Test for inhibition of adaptive immune response in ovalbumin-induced asthma/chronic obstructive pulmonary disease model

### 6-1: Inhibitory effect against production of T cell-derived cytokines isolated from lung tissue

In order to examine whether the extract of the present invention has the effect of inhibiting adaptive immune responses in an ovalbumin (OVA)-induced asthma/chronic obstructive pulmonary disease model, the content of T cell-derived cytokines isolated from lung tissue was measured (see Seong Gyu Jeon et al., J. Allergy Clin. Immunol., 2007, 119, 831-837).

Specifically, a mixture of 75 *µ*g of ovalbumin (OVA, Sigma) and 2 mg of aluminum hydroxide (Alum, Sigma) was intraperitoneally administered twice (days 0 and 7) into BALB/c female mice (6 weeks old; provided from Animal Laboratory in Biotech Center, Pohang University of Science & Technology) to sensitize the mice. At 14, 15 and 21 days after the first sensitization date, 50 *µ*g of ovalbumin in PBS was challenged into the nasal cavity to induce asthma and chronic obstructive pulmonary disease. In addition, at 14, 15 and 21 days at which ovalbumin was challenged, each of 1.0% CMC, 200 mg/10 mℓ/kg of the extract of Example 1, 1 mg/10 mℓ/kg of the comparative drug dexamethason and 10 mg/10 mℓ/kg of the comparative drug montelukast was orally administered twice a day. At 6 hours after the final challenge, the mice were anesthetized with ketamine and xylazine, and the lung tissue was extracted from the mice.

Then, the lung tissue was finely cut with a blade and incubated with 5 ml of 1X Trypsin-EDTA and 1000 units of chollagenase at 37 °C for 10 immunes. Then, the resulting tissue was passed through a 100 *µ*m mesh to collect only immune cells. In order to stimulate only T cells among the obtained cells, 1×10⁵ cells and anti-CD28 (1 *µ*g/mℓ) were added to a 96-well plate coated with anti-CD3 (1 *µ*g/mℓ) and were incubated in an incubator at 37 °C for 12 hours. Then, the supernatant was collected, and the amount of cytokine IL-4 (interleukin-4) in the supernatant was measured using an ELISA (enzyme-linked immunosorbent assay). The results of the measurement are shown in Table 7 below.

**Table 7**

| Conditions | Negative control group (ovalbumin) | Positive control group (ovalbumin + aluminum hydroxide) | Dexamethason (1 mg/kg *b.i.d.*) | Montelukast (10 mg/kg *b.i.d*.) | Extract of Example 2 (200 mg/kg *b.i.d.*) |
|---|---|---|---|---|---|
| IL-4 inhibition rate (%) | 82.74 | 0.00 | 43.57 | 14.07 | 37.29 |

As can be seen in Table 7 above, the extract (200 mg/kg) of Example 2 significantly inhibited the production of IL-4, and this inhibitory effect was similar to that of the comparative drug dexamethason.

### 6-2: Test for inhibition of expression of co-stimulatory molecule in dendritic cells isolated from lung tissue

In order to examine whether the extract of the present invention has the effect of inhibiting the expression of a co-stimulatory molecule in dendritic cells isolated from the lung tissue of an ovalbumin-induced asthma/chronic obstructive pulmonary disease model, the following test was performed.

Specifically, a portion of the lung tissue obtained in Test Example 6-1 was cut finely with a blade, and then incubated with 5 ml of 1×Trypsin-EDTA and 1000 units of collagenase at 37 °C for 10 minutes. Then, the tissue was passed through a 100 *µ*m mesh to collect only immune cells, and the cells were double-stained with each of the dendritic cell marker CD11c and the co-stimulatory molecules CD40, CD80, CD86 and MHCII (major histocompatibility complex II). Next, the expression levels of the co-stimulatory molecules in the dendritic cells were measured using FACS (fluorescence activated cell sorter), and the results of the measurement are shown in FIG. 2.

As can be seen in FIG. 2, the expression levels of the four co-stimulatory molecules in the group administered with the extract (200 mg/kg b.i.d) of Example 2 all decreased, similar to those in the groups administered with the comparative drugs dexamethason and montelukast.

### Test Example 7: Examination of anti-asthma effect and inhibitory effect against adaptive immune responses in LPS-induced asthma/chronic obstructive pulmonary disease model

### 7-1: Test for Inhibition of inflammatory cells in pulmonary bronchoalveolar lavage

Whether the extract of the present invention has the effect of inhibiting pulmonary bronchial inflammation in an LPS (lipopolysacoharide) -induced asthma/chronic obstructive pulmonary disease model was examined (see You-Sun Kim et al., J. Immunol., 2010, 185, 5648-5655; Jun-Pyo Choi et al., Allergy, 2010, 65, 1322-1330).

Specifically, a mixture of 75 *µ*g of ovalbumin and 10 *µ*g of LPS (Sigma) was intranasally administered a total of four times (days 0, 1, 2 and 7) into C57BL/6 male mice (6 weeks old; provided from the Animal Laboratory in Biotech Center, Pohang University of Science & Technology) to sensitize the mice. At 14, 15, 21 and 22 days from the first sensitization date, 50 *µ*g of ovalbumin in PBS was challenged into the nasal cavity to induce asthma and chronic obstructive pulmonary disease. In addition, at 14, 15, 21 and 22 days at which challenge was performed, each of 1.0% CMC, 100, 200 and 400 mg/10 mℓ/kg of the extract of Example 2, 1 mg/10 mℓ/kg of the comparative drug dexamethason and 10 mg/10 mℓ/kg of the comparative drug montelukast was orally administered twice a day. At 48 hours after the final challenge, the mice were anesthetized by intraperitoneal administration of ketamine and xylazine, and the organ of the mice was exposed and a catheter was inserted therein. Then, the bronchoalveoli were laved with each of 0.8 and 0.7 mℓ of sterile phosphate buffered saline to obtain a bronchoalveolar lavage. The bronchoalveolar lavage was centrifuged at 4 °C at 1000 rpm for 5 minutes, and the supernatant was removed. 2 ml of 10% FBS-IMDM (fetal bovine serum - Iscove's Modified Dulbecco's Medium) was added to the cells which were then counted. Then, the cells were cytospun, plated on a slide, and Diff-Quick stained. At least 300 inflammatory cells were observed by the staining method and classified into eosinophils, macrophages, neutrophils and lymphocytes, and the cell numbers of the groups treated with dexamethason, montelukast and the extract of Example 2 were compared with the ratios of inflammatory cells in the pulmonary bronchoalveolar lavages of the negative control group and the positive control group, thereby evaluating the inhibitory effects of the test materials against airway inflammation. The results of the evaluation are shown in Table 8 below.

**Table 8**

| Conditions | Inhibition rate(%) | | | | | | |
|---|---|---|---|---|---|---|---|
| | Negative control (ovalbumin) | Positive control (ovalbumin + LPS) | Dexamethason (1 mg/kg *b.i.d.*) | Montelukast (10 mg/kg *b.i.d.*) | Extract of Example 2 | | |
| | | | | | 100 mg/kg *b.i.d.* | 200 mg/kg *b.i.d.* | 400 mg/kg *b.i.d.* |
| Total cells | 90.1 | 0.0 | 40.4 | 10.6 | 15.2 | 44.7 | 44.7 |
| Macrophages | 79.5 | 0.0 | 27.3 | 8.2 | 10.3 | 18.1 | 58.9 |
| Eosinophils | 99.7 | 0.0 | 51.1 | 13.3 | 39.0 | 65.7 | 30.2 |

As can be seen in Table 8 above, dexamethason had only a slight inhibitory effect against the invasion of eosinophils, and montelukast had a low inhibitory effect against the invasion of all types of cells, whereas the extract of Example of the present invention strongly inhibited the invasion of macrophages and eosinophils (45% reduction in the groups administered with 200 and 400 mg/kg *b. i. d.* of the extract).

### 7-2: Test for inhibition of invasion of inflammatory cells into lung tissue

Whether the extract of the present invention has the effect of inhibiting the invasion of inflammatory cells into lung tissue in an LPS-induced asthma/chronic obstructive pulmonary disease model was evaluated.

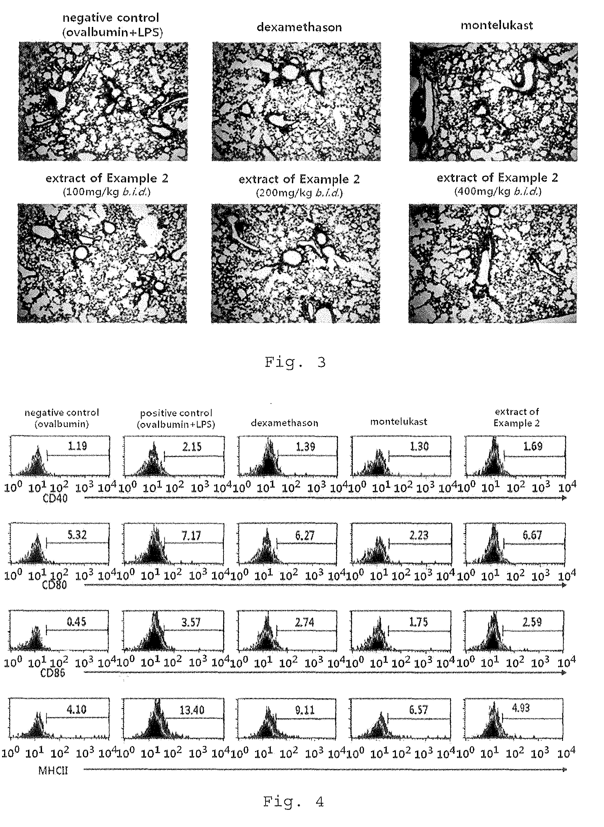

Specifically, after collecting the pulmonary bronchoalveolar lavage in Test Example 7-1, 10% formalin solution was injected into the lung through a catheter. Then, the lung was isolated, embedded with paraffin, sectioned to a thickness of 4 mm, and stained with H&E (hematoxylin-eosin). The invasion of inflammatory cells into a portion surrounding the airway was measured (see FIG. 3), and the inflammations of the airway and the pulmonary parenchyma were compared and scored. The results are shown in Table 9 below.

**Table 9**

| Inhibition rate (%) | Negative control (ovalbumin) | Positive control (ovalbumin + LPS) | Dexa. | Mona. | Extract of Example 2 | | |
|---|---|---|---|---|---|---|---|
| | | | Dexamethason (1 mg/kg *b.i.d.*) | Montelukast (10 mg/kg *b.i.d.*) | 100 mg/kg *b.i.d.* | 200 mg/kg *b.i.d.* | 400 mg/kg *b.i.d.* |
| Inhibition rate (%) | 76.29 | 0.0 | 28.87 | 8.25 | 15.98 | 38.14 | 44.02 |

As can be seen in Table 9 above, the invasion of inflammatory cells in the pulmonary bronchi of the groups administered orally with 200 and 400 mg/kg *b.i.d.* of the extract of Example 2 significantly decreased. Meanwhile, the group administered with dexamethason showed a decrease in inflammation cell invasion of 28.87%, and montelukast showed no special effect.

### 7-3: Test for inhibition of inflammatory mediators in bronchoalveolar lavage

In order to examine whether the extract of the present invention has the effect of inhibiting inflammatory mediators in a bronchoalveolar lavage in an LPS-induced asthma/chronic obstructive pulmonary disease model, the expression pattern of the inflammatory mediator IP-10 (interferon-gamma inducible protein-10) in the bronchoalveolar lavage obtained in Test Example 7-2 was measured using an ELISA assay, and the results of the measurement are shown in Table 10 below.

**Table 10**

| Conditions | Negative control (ovalbumin) | Positive control (ovalbumin + LPS) | Dexamethason (1 mg/kg *b.i.d.*) | Montelukast (10 mg/kg *b.i.d.*) | Extract of Example 2 | | |
|---|---|---|---|---|---|---|---|
| | | | | | 100 mg/kg *b.i.d.* | 200 mg/kg *b.i.d.* | 400 mg/kg *b.i.d.* |
| IP-10 inhibition rate (%) | 91.59 | 0.00 | 4.09 | 1.80 | 38.56 | 38.94 | 44.33 |

As can be seen in Table 10 above, all the groups administered with the extract of Example 2 of the present invention showed a decrease in IP-10 production of about 38-44%. However, dexamethason and montelukast had little or no effects.

### 7-4: Test for the ability to produce antigen-specific antibody

In order to examine whether the extract of the present invention has the ability to produce an antigen-specific antibody in an LPS-induced asthma/chronic obstructive pulmonary disease model, the LPS-induced asthma/chronic obstructive pulmonary disease model was prepared by treatment with each of the extract of Example 2 and a comparative drug in the same manner as described in Test Example 7-1. At 48 hours after the final challenge, the mice were anesthetized by intraperitoneal injection with ketamine and xylazine. Then, blood was collected from the heart using a 1 mℓ syringe, and serum was separated from the blood by centrifugation. Then, an anti-ovalbumin-specific IgG1 antibody in the serum was measured according to an ELISA assay, and the results of the measurement are shown in Table 11 below.

**Table 11**

| Conditions | Negative control (ovalbumin) | Positive control (ovalbumin + LPS) | Dexamethason (1 mg/kg *b.i.d.*) | Montelukast (10 mg/kg *b.i.d.*) | Extract of Example 2 | | |
|---|---|---|---|---|---|---|---|
| | | | | | 100 mg/kg *b.i.d.* | 200 mg/kg *b.i.d.* | 400 mg/kg *b.i.d.* |
| IGg1 in serum | 13.70 | 67.86 | 28.94 | 44.84 | 20.74 | 23.60 | 27.45 |
| Inhibition rate (%) | 79.82 | 0.00 | 57.35 | 33.91 | 69.44 | 65.22 | 59.54 |

As can be seen in Table 11 above, the group administered with the extract of Example 2 showed an inhibition rate of 65-70% at doses of 100 and 200 mg/kg *b.i.d*., and the inhibitory effect was superior to that of dexamethason or montelukast.

### 7-5: Test for inhibition of production of T cell-derived cytokine isolated from lung tissue

In order to examine whether the extract of the present invention has the effect of inhibiting T cell-derived cytokine isolated from the lung tissue of an LPS-derived asthma/chronic obstructive pulmonary disease model, the following test was performed.

Specifically, the LPS-induced asthma/chronic obstructive pulmonary disease model was prepared by treatment with each of the extract of Example 2 and a comparative drug in the same manner as described in Test Example 7-1. At 6 hours after the final challenge, the mice were anesthetized by intraperitoneal injection with ketamine and xylazine, and lung tissue was obtained from the mice. The lung tissue was cut finely with a blade and incubated with 5 ml of 1X Trypsin-EDTA and 1000 units of chollagenase at 37 °C for 10 minutes. Then, the tissue was passed through a 100 *µ*m mesh to collect only immune cells. To stimulate T cells among the collected cells, 1×10⁵ cells and anti-CD28 (1 *µ*g/mℓ) were added to a 96-well plate coated with anti-CD3 (1 *µ*g/mℓ) and were incubated in an incubator at 37 °C for 12 hours. Next, the supernatant was collected, and the amounts of the cytokines IL-17 (interleukin 17) and IFN-γ (interferon-γ) in the supernatant were measured using an ELISA (enzyme-linked immunosorbent assay). The results of the measurement are shown in Table 12 below.

**Table 12**

| Conditions | Inhibition rate (%) | | | | |
|---|---|---|---|---|---|
| | Negative control (ovalbumin) | Positive control (ovalbumin + LPS) | Dexamethason (1 mg/kg *b.i.d.*) | Montelukast (10 mg/kg *b.i.d.*) | Extract of Example 2 (400 mg/kg *b.i.d.*) |
| IL-17 inhibition rate (%) | 74.16 | 0.00 | 23.12 | 68.42 | 64.92 |
| IFN-γ inhibition rate (%) | 99.66 | 0.00 | 15.19 | 14.50 | 87.81 |

As can be seen in Table 12 above, the extract of Example 2 significantly inhibited IL-17 and IFN-γ compared to dexamethason or montelukast.

### 7-6: Test for inhibition of expression of co-stimulatory molecule in dendritic cells isolated from lung tissue

In order to examine whether the extract of the present invention has the effect of inhibiting the expression of co-stimulatory molecules in dendritic cells isolated from lung tissue in an LPS-induced asthma/chronic obstructive pulmonary disease model, the following test was performed.

Specifically, a portion of the lung tissue obtained in Test Example 7-5 was cut finely with a blade, and then incubated with 5 ml of 1×Trypsin-EDTA and 1000 units of collagenase at 37 °C for 10 minutes. Then, the tissue was passed through a 100 *µ*m mesh to collect only immune cells, and the cells were double-stained with each of the dendritic cell marker CD11c and the co-stimulatory molecules CD40, CD80, CD86 and MHCII (major histocompatibility complex II). Next, the expression levels of the co-stimulatory molecules in the dendritic cells were measured using FACS (fluorescence activated cell sorter), and the results of the measurement are shown in FIG. 4.

As can be seen in FIG. 4, the extract of Example 2 inhibited the expressions of the co-stimulatory molecules CD40, CD80, CD86 and MHCII, and particularly, the inhibitory effect of the extract of Example 2 against MHCII was superior to that of dexamethason or montelukast.

### Test Example 8: Test for anti-asthma effect and inhibitory effect against adaptive immune responses in E. coli-BV-induced asthma/chronic obstructive pulmonary disease model

### 8-1: Test for inhibition of inflammatory cells in pulmonary bronchoalveolar lavage

Whether the extract of the present invention has the effect of inhibiting pulmonary bronchial inflammation in an *E*. *coli*-EV-induced asthma/chronic obstructive pulmonary disease model was evaluated.

Specifically, a mixture of 10 ng of *E*. *coli*-EV and PBS was intranasally administered a total of four times (days 0, 1, 2 and 7) into C57BL/6 male mice (6 weeks old; provided from the Animal Laboratory in Biotech Center, Pohang University of Science & Technology) to sensitize the mice. At 14, 15, 21 and 22 days from the first sensitization date, 10 ng of *E*. *coli*-EV was challenged into the nasal cavity to induce asthma and chronic obstructive pulmonary disease. In addition, at 14, 15, 21 and 22 days at which the challenge was performed, each of 1.0% CMC, 100, 200 and 400 mg/10 mℓ/kg of the extract of Example 2, 1 mg/10 mℓ/kg of the comparative drug dexamethason and 10 mg/10 mℓ/kg of the comparative drug montelukast was orally administered twice a day. At 48 hours after the final challenge, the mice were anesthetized by intraperitoneal administration of ketamine and xylazine, and the organ of the mice was exposed and a catheter was inserted therein. Then, the bronchoalveoli were laved with each of 0.8 and 0.7 mℓ of sterile phosphate buffered saline to obtain a bronchoalveolar lavage. The bronchoalveolar lavage was centrifuged at 4 °C at 1000 rpm for 5 minutes, and the supernatant was removed. 2 ml of 10% FBS-IMDM (fetal bovine serum - Iscove's Modified Dulbecco's Medium) was added to the cells which were then counted. Then, the cells were cytospun, plated on a slide, and Diff-Quick stained. At least 300 inflammatory cells were observed by the staining method and classified into eosinophils, macrophages, neutrophils and lymphocytes, and the cell numbers of the groups treated with dexamethason, montelukast and the extract of Example 2 were compared with the ratios of inflammatory cells in the pulmonary bronchoalveolar lavages of the negative control group and the positive control group, thereby evaluating the inhibitory effects of the test materials against airway inflammation. The results of the evaluation are shown in Table 13 below.

**Table 13**

| Conditions | Inhibition rate(%) | | | | | | |
|---|---|---|---|---|---|---|---|
| | Negative control (PBS) | Positive control (E. coli-EV) | Dexamethason (1 mg/kg *b.i.d.*) | Montelukast (10 mg/kg *b.i.d.*) | Extract of Example 2 | | |
| | | | | | 100 mg/kg *b.i.d.* | 200 mg/kg *b.i.d.* | 400 mg/kg *b.i.d.* |
| Total cells | 92.7 | 0.0 | 9.2 | 1.2 | 14.0 | 16.3 | 23.4 |
| Macrophages | 84.4 | 0.0 | 24.7 | 1.3 | 25.7 | 21.4 | 32.1 |
| Eosinophils | 99.5 | 0.0 | 1.1 | 15.4 | 7.7 | 31.7 | 35.1 |

As can be seen in Table 13 below, the extract of Example 2 inhibited the production of various immune cells in the *E*. *coli*-EV-induced asthma/chronic obstructive pulmonary disease model, and this inhibitory effect was superior to that of dexamethason or montelukast.

### 8-2: Test for inhibition of inflammatory mediators in bronchoalveolar lavage

The expression patterns of the inflammatory mediators MCP-1 (monocyte chemoattractant protein-1) and MIP-1α (macrophage inflammatory protein-1α) in the bronchoalveolar lavage obtained in Test Example 8-1 were measured using an ELISA assay, and the results of the measurement are shown in Table 14 below.

**Table 14**

| Conditions | Inhibition rate(%) | | | | | | |
|---|---|---|---|---|---|---|---|
| | Negative control (PBS) | Positive control (E. coli-EV) | Dexamethason (1 mg/kg *b.i.d.*) | Montelukast (10 mg/kg *b.i.d.*) | Extract of Example 2 | | |
| | | | | | 100 mg/kg *b.i.d.* | 200 mg/kg *b.i.d.* | 400 mg/kg *b.i.d.* |
| MCP-1 | 82.2 | 0.0 | 1.2 | 11.5 | 43.2 | 55.0 | 56.1 |
| MIP-1α | 83.3 | 0.0 | 8.3 | 4.2 | 25.0 | 41.7 | 45.8 |

As can be seen in Table 14 above, the extract of Example 2 inhibited the production of MCP-1 and MIP-1α in a dose-dependent manner. However, dexamethason and montelukast had little or no inhibitory effects.

### 8-3: Test for the ability to produce antigen-specific antibody

In order to examine whether the extract of the present invention has the ability to produce an antigen-specific antibody in an *E*. *coli*-EV-induced asthma/chronic obstructive pulmonary disease model, the *E*. *coli*-EV-induced asthma/chronic obstructive pulmonary disease model was prepared by treatment with each of the extract of Example 2 and a comparative drug in the same manner as described in Test Example 8-1. At 48 hours after the final challenge, the mice were anesthetized by intraperitoneal injection with ketamine and xylazine. Then, blood was collected from the heart using a 1 mℓ syringe, and serum was separated from the blood by centrifugation. Then, anti-*E*. *coli*-EV-specific IgG1 and IgG2a antibodies in the serum were measured according to an ELISA assay, and the results of the measurement are shown in Table 15 below.

**Table 15**

| Conditions | Inhibition rate(%) | | | | | | |
|---|---|---|---|---|---|---|---|
| | Negative control (PBS) | Positive control (E. coli-EV) | Dexamethason (1 mg/kg *b.i.d.*) | Montelukast (10 mg/kg *b.i.d.*) | Extract of Example 2 | | |
| | | | | | 100 mg/kg *b.i.d.* | 200 mg/kg *b.i.d.* | 400 mg/kg *b.i.d.* |
| IgG1 | 100.0 | 0.0 | 93.8 | 59.7 | 59.9 | 92.9 | 93.8 |
| IgG2a | 100.0 | 0.0 | 55.7 | 27.2 | 65.8 | 69.3 | 98.4 |

As can be seen in Table 15 above, the extract of Example 2 had the effect of significantly inhibiting both IgG1 and IgG2a, and this effect was excellent compared to that of dexamethason or montelukast.

### 8-4: Test for inhibition of production of T cell-derived cytokine isolated from lung tissue

In order to examine whether the extract of the present invention has the effect of inhibiting T cell-derived cytokine isolated from the lung tissue of an E. coli-EV-derived asthma/chronic obstructive pulmonary disease model, the following test was performed.

Specifically, the LPS-induced asthma/chronic obstructive pulmonary disease model was prepared by treatment with each of the extract of Example 2 and a comparative drug in the same manner as described in Test Example 8-1. At 6 hours after the final challenge, the mice were anesthetized by intraperitoneal injection with ketamine and xylazine, and lung tissue was obtained from the mice. The lung tissue was cut finely with a blade and incubated with 5 ml of 1X Trypsin-EDTA and 1000 units of chollagenase at 37 °C for 10 minutes. Then, the tissue was passed through a 100 *µ*m mesh to collect only immune cells. To stimulate T cells among the collected cells, 1×10⁵ cells and anti-CD28 (1 *µ*g/mℓ) were added to a 96-well plate coated with anti-CD3 (1 *µ*g/mℓ) and were incubated in an incubator at 37 °C for 12 hours. Next, the supernatant was collected, and the amounts of cytokines in the supernatant were measured using an ELISA assay. The results of the measurement are shown in Table 16 below.

**Table 16**

| Conditions | Inhibition rate (%) | | | | |
|---|---|---|---|---|---|
| | Negative control (PBS) | Positive control (E. coli-EV) | Dexamethason (1 mg/kg *b.i.d.*) | Montelukast (10 mg/kg *b.i.d.*) | Extract of Example 2 (200 mg/kg *b.i.d.*) |
| IL-17 inhibition rate (%) | 100.00 | 0.00 | 1.12 | 0.22 | 43.07 |
| IFN-γ inhibition rate (%) | 55.79 | 0.00 | 1.13 | 0.23 | 47.34 |

As can be seen in Table 16 above, dexamethason and montelukast had little or no inhibitory effects against the production of IL-17 and IFN-γ, whereas the extract (200 mg/kg *b.i.d.*) of Example 2 significantly inhibited the production of IL-17 and IFN-γ.

### 8-5: Test for inhibition of expression of co-stimulatory molecule in dendritic cells isolated from lung tissue

In order to examine whether the extract of the present invention has the effect of inhibiting the expression of co-stimulatory molecules in dendritic cells isolated from the lung tissue of an *E*. *coli*-EV-induced asthma/chronic obstructive pulmonary disease model, the following test was performed.

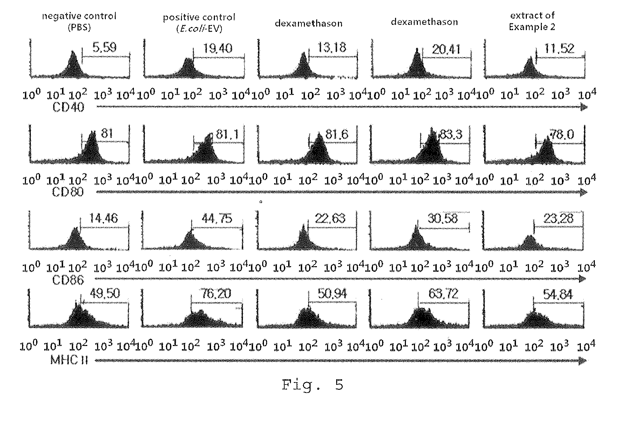

Specifically, a portion of the lung tissue obtained in Test Example 8-4 was cut finely with a blade, and then incubated with 5 ml of 1×Trypsin-EDTA and 1000 units of collagenase at 37 °C for 10 minutes. Then, the tissue was passed through a 100 *µ*m mesh to collect only immune cells, and the cells were double-stained with each of the dendritic cell marker CD11c and the co-stimulatory molecules CD40, CD80, CD86 and MHCII (major histocompatibility complex II). Next, the expression levels of the co-stimulatory molecules in the dendritic cells were measured using FACS (fluorescence activated cell sorter), and the results of the measurement are shown in FIG. 5.

The expression levels of the co-stimulatory molecules in the dendritic cells (antigen-presenting cells) were measured. As a result, as can be seen in FIG. 5, the groups treated with dexamethason and montelukast showed a decrease in the expression of CD86 and MHCII, and the group treated with the extract (200 mg/kg *b.i.d.*) of Example 2 showed decreases in the expression levels of all CD40, CD80, CD86 and MHCII.

### Test Example 9: Test for expectorant activity

Using the method of Engler et al. (Engler H, Szelenyi I, J. Pharmacol. Moth., 11, 151∼157, 1984; Jian-Hua Shang et al, J. Ethnopharmacology, 129, 293∼298, 2010), the expectorant activity of the extract of the present invention was measured.

Specifically, as experimental animals, male ICR mice (weighed 30-33 g; Orientbio, Korea) were divided into groups, each consisting of 10 mice. Each of the extract of Example 2, 250 mg/kg of the comparative drug ambroxol (Sigma) and 500 mg/kg of ammonium chloride was administered intraperitoneally into the mice which have been fasted for one day. After 30 minutes, 5% phenol red was administered intraperitoneally into the mice. After 30 minutes, the mice were subjected to cervical dislocation, and blood was discharged from the abdominal aorta, after which the trachea was completely excised. The isolated trachea was cold-stored in 1 mℓ of physiological saline. After 24 hours, the cold-stored trachea was centrifuged at 3000 rpm for 10 minutes, and 1N caustic soda (NaOH) was added to the supernatant in an amount of 0.1 mℓ based on 1 mℓ of the supernatant, after which the absorbance at 546 nm was measured, thereby measuring the expectorant activity of the test material as the concentration of phenol red. The results of the measurement are shown in Table 17 below.

**Table 17**

| Conditions | | Oral dose (mg/kg) | Expectorant activity (%) |
|---|---|---|---|
| Example 2 | | 100 | 10 |
| | | 200 | 24 |
| | | 400 | 29 |
| Comparative drugs | Ambroxol | 250 | 27 |
| | Magnesium chloride | 500 | 33 |

As can be seen in Table 17 above, the extract of the present invention showed excellent expectorant activity in a dose-dependent manner. Particularly, the expectorant activity was the highest in the group administered with 400 mg/kg of the extract.

### Test Example 10: Test for antitussive activity

In order to evaluate the antitussive activity of the extract of the present invention (American Journal of Respiratory and Critical Care Medicine, 1998, 158[1], 42-48), the extract of Example 2 was administered into guinea pigs, and then citric acid was sprayed into the guinea pig to induce coughing, after which the number of coughs was measured. Citric acid is frequently used in studies on the activity of airway sensory nerves, and the inhalation of citric acid causes bronchoconstriction, nasal stimulation, coughing, bronchial hypersensitivity and the like.

Specifically, as experimental animals, Hartely male guinea pigs (weighed 350-400 g; Orientbio, Korea) were divided into groups, each consisting of 10-12 animals. Each of the extract of Example 2 and 50 mg/kg of the comparative drug theobromine (Sigma) was administered orally into the grouped mice which have been fasted for one day. After 1 hour, 0.3M citric acid as a cough inducer was sprayed into the animals to induce coughing. The number of coughs generated for 15 minutes from the start of exposure to the cough inducer was measured using Whole Body Plethysmometer (BUXCO), and the inhibition rate (%) of coughing was calculated relative to a negative control group (administered with 1% CMC). The results of the calculation are shown in Table 18 below.

| Conditions | | Oral dose (mg/kg) | Cough inhibition rate (%) |
|---|---|---|---|
| Example 2 | | 100 | 10 |
| | | 200 | 49 |
| | | 400 | 48 |
| Comparative drug | theobromine | 50 | 36 |

As can be seen in Table 18 above, the effect of inhibiting citric acid-induced coughing in the guinea pig animal model was higher in the groups administered with 200 and 400 mg/kg of the extract of Example 2 than in the group administered with the comparative drug theobromine.

### Test Example 11: Toxicity test

### 11-1: Acute toxicity

In order to examine toxicity that occurs when the extract of Example 2 of the present invention is administered once into Sprague-Dawley (SD) rats, the following test was performed. The extract of Example 2 was administered once into each rat group (consisting of 5 males and 5 female) at a dose of each of 1,250, 2,500 and 5,000 mg/kg, and then lethality, general symptoms, weight and necroscopy findings were observed and compared with those of a vehicle control group.

As a result, no dead animal was observed during the test period, and an abnormal change in weight associated with the administration of the test material was not observed. In addition, no abnormal necroscopy finding was observed.

From the above results, it could be seen that the extract of Example 2 of the present invention showed a minimum lethal dose ((mℓ D) of more than 5, 000 mg/kg when administered orally once into Sprague-Dawley male and female rats, suggesting that the extract is a safe material.

### 11-2: Test for toxicity of repeated administration

The extract of Example 2 of the present invention was repeatedly administered orally over 2 weeks in order to examine the toxicity of the extract. Specifically, Sprague-Dawley male and female rats were divided into the following groups: groups administered with the test material at doses of 1,000, 2,000 and 4,000 mg/kg/day, respectively; and a vehicle control group administered with a vehicle alone. Each animal group consisted of 5 males and 5 females. Test items included lethality, general symptoms, a change in weight, the intake of feed and water, urine examination, hematological and hematobiochemical examination, necroscopy findings, organic weight and histopathological findings.

As a result, a dead animal associated with the toxicity of the test material was not observed during the test period, and a change in general symptoms associated with the administration of the test material was not observed. In addition, a significant change in the intake of feed and water, caused by the administration of the test material, was not observed.

In the results of urine examination, abnormal toxicological findings associated with the administration of the test material were not observed. In the results of hematological and hemato-biochemical examination, abnormal toxicological findings associated with the administration of the test material were not observed. In addition, in the results of observation of organic weight and visual necroscopy findings, abnormal visual findings associated with the administration of the test material were not observed. In the results of histopathological observation, abnormal toxicological findings associated with the administration of the test material were not observed.

As described above, the extract of Example 2 of the present invention was repeatedly administered into SD rats over 2 weeks, and as a result, it could be seen that the no-observed-adverse-effect-level (NOAEL) of the extract was 4,000 mg/kg/day in both the males and the females, suggesting that the extract is a safe material.

### Formulation Example 1: Preparation of tablet

| | |
|---|---|
| Mixed extract of Example 2 | 200 mg |
| Corn starch | 50 mg |
| Cellactose | 100 mg |
| Crospovidone | 20 mg |
| Light anhydrous silicic acid | 5 mg |
| Lactose | 70 mg |
| Magnesium stearate | 5 mg |

The above components were mixed with each other, and the mixture was compressed according to a conventional tablet preparation method, thereby preparing a tablet.

### Formulation Example 2: Preparation of powder

| | |
|---|---|
| Mixed extract of Example 2 | 200 mg |
| Corn starch | 50 mg |
| lactose | 100 mg |
| Talc | 10 mg |

The above components were mixed with each other, and the mixture was filled into an airtight packet according to a conventional powder preparation method, thereby preparing a powder formulation.

### Formulation Example 3: Preparation of granule

| | |
|---|---|
| Mixed extract of Example 2 | 300 mg |
| Corn starch | 1000 mg |
| Lactose | 1000 mg |
| Enzymatically treated stevia | 50 mg |
| Talc | 10 mg |

The above components were mixed with each other, and the mixture was filled into an airtight packet according to a conventional granule preparation method, thereby preparing a granule formulation.

### Formulation Example 4: Preparation of capsule

| | |
|---|---|
| Mixed extract of Example 2 | 300 mg |
| Microcrystalline cellulose | 150 mg |
| Lactose | 30 mg |
| Magnesium stearate | 0.5 mg |

The above components were mixed with each other, and the mixture was filled into a gelatin packet according to a conventional capsule preparation method, thereby preparing a capsule formulation.

### Formulation Example 5: Preparation of injectable formulation

| | |
|---|---|
| Mixed extract of Example 2 | 50 mg |
| Mannitol | 100 mg |
| Sterile distilled water for injection | 3500 mg |
| Na₂HPO₄, 12H₂O | 20 mg |
| pH adjusting agent | q.s. |

According to a conventional method for preparation of an injectable formulation, the active ingredient and other components were dissolved in sterile distilled water, and the solution was adjusted to a pH of about 7.5. Then, the solution was filled into a 2 ml ampoule with distilled water for injection and sterilized, thereby preparing an injectable formulation.

### Formulation Example 6: Preparation of liquid formulation

| | |
|---|---|
| Mixed extract of Example 2 | 500 mg |
| Isomerized sugar | 10 g |
| Mannitol | 5 g |
| Purified water | q.s. |

According to a conventional method for preparation of a liquid formulation, the above components were dissolved in purified water, and orange micron was added thereto and stirred, and then purified water was added thereto to a total volume of 100 ml. Then, the solution was filled into a container and sterilized, thereby preparing a liquid formulation.

### Formulation Example 6: Preparation of health functional food

| | |
|---|---|
| Mixed extract of Example 2 | 500 mg |
| Vitamin mixture | q.s. |
| Vitamin A acetate | 70 *µ*g |
| Vitamin E | 1.0 mg |
| Vitamin B1 | 0.13 mg |
| Vitamin B2 | 0.15 mg |
| Vitamin B6 | 0.5 mg |
| Vitamin B12 | 0.2 *µ*g |
| Vitamin C | 10 mg |
| Biotin | 10 *µ*g |
| nicotinic acid amide | 1.7 mg |
| Folic acid | 50 *µ*g |
| Calcium pantotenate | 0.5 mg |
| Mineral mixture | q.s. |
| Ferrous sulfate | 1.75 mg |
| Zinc oxide | 0.82 mg |
| Magnesium carbonate | 25.3 mg |
| Potassium phosphate monobasic | 15 mg |
| Calcium phosphate dibasic | 55 mg |
| Potassium citrate | 90 mg |
| Calcium carbonate | 100 mg |
| Magnesium chloride | 24.8 mg |

Although the composition ratio of the above vitamins and mineral mixture is a preferred ratio suitable for health functional foods, it may be optionally changed. According to a conventional method for preparation of health functional foods, the above components were mixed with each other, and the mixture was granulated. The granule formulation can be used for the preparation of a health functional food according to a conventional method.

### Formulation Example 8: Preparation of health functional health beverage

| | |
|---|---|
| Mixed extract of Example 2 | 200 mg |
| Citric acid | 1000 mg |
| Oligosaccharide | 100 g |
| Plum concentrate | 2 g |
| Taurin | 1 g |
| Purified water | To 900 ml |

According to a conventional method for preparation of health functional beverages, the above components were mixed with each other, and the mixture was heated with stirring at 85 °C for about 1 hour. Then, the solution was filtered, sealed in a sterilized 2ℓ container, sterilized, and then cold-stored. The cold-stored material was used for the preparation of the inventive health functional beverage composition.

The above composition ratio is a preferable ratio suitable for favorite beverages, but it may be optionally changed depending on regional and national preferences, depending on the user, nations and the intended use.

## Claims

1. A composition for preventing or treating respiratory disease, which contains, as an active ingredient, an extract obtained by extracting a mixture of Cnidii rhizoma and Polygoni cuspidati radix with a C₁ to C₄ lower alcohol or a 10-95% aqueous solution of the alcohol.

2. The composition of claim 1, wherein the extract is an extract solubilized with a solvent selected from the group consisting of ethyl acetate, dichloromethane and butanol.

3. The composition of claim 1 or 2, wherein the mixing weight ratio between Cnidii rhizoma and Polygoni cuspidati radix is 0.1:1 to 10:1 (Cnidii rhizoma: Polygoni cuspidati radix).

4. The composition of claim 3, wherein the mixing weight ratio between Cnidii rhizoma and Polygoni cuspidati radix is 0.5:1 to 5:1.

5. The composition of claim 1 or 2, wherein the respiratory disease is selected from the group consisting of asthma, chronic obstructive pulmonary disease, allergic rhinitis, pyothrox, lung abscess, coughing, phlegm, bronchitis, laryngopharyngitis, tonsillitis and laryngitis.

6. The composition of claim 5, wherein the asthma is selected from the group consisting of bronchial asthma, atopic asthma, atopic bronchial IgE-mediated asthma, non-atopic asthma, allergic asthma and non-allergic asthma.

7. The composition of claim 5, wherein the bronchitis is selected from the group consisting of acute bronchitis, chronic bronchitis, bronchiolitis, catarrhal bronchitis, and obstructive and inflammatory bronchial diseases.

8. A health functional food for preventing or alleviating respiratory disease, which contains, as an active ingredient, an extract obtained by extracting a mixture of Cnidii rhizoma and Polygoni cuspidati radix with a C₁ to C₄ lower alcohol or a 10-95% aqueous solution of the alcohol.

9. The health functional food of claim 8, wherein the extract is an extract solubilized with a solvent selected from the group consisting of ethyl acetate, dichloromethane and butanol.
